# EUROPÄISCHE PATENTSCHRIFT

(11) **EP 4 204 406 B1**
(45) Veröffentlichungstag und Bekanntmachung des Hinweises auf die Patenterteilung: **18.02.2026**
(21) Anmeldenummer: 21763295.9
(22) Anmeldetag: 12.08.2021
(51) Int. Cl.: C07D 319/08, C11B 9/00

(54) **ANREICHERUNG EINES DIASTEREOMERS IN MAGNOLAN**
ENRICHMENT OF A DIASTEREOMER IN MAGNOLAN
ENRICHISSEMENT DE MAGNOLANE PAR UN DIASTÉRÉO-ISOMÈRE

(30) Priorität: 25.08.2020 WO PCT/EP2020/073775
(43) Veröffentlichungstag der Anmeldung: 05.07.2023
(73) Patentinhaber: Symrise AG, 37603 Holzminden (DE)
(72) Erfinder: EH, Marcus, 37603 Holzminden (DE); BORCHERT, Susanne, 37603 Holzminden (DE); BETZER, Marcus, 37603 Holzminden (DE); ILLAN, Alexandre, 37603 Holzminden (DE); HEPPEKAUSEN, Johannes, 37603 Holzminden (DE)
(74) Vertreter: Global IP Europe Patentanwaltskanzlei
(86) Internationale Anmeldenummer: PCT/EP2021/072541
(87) Internationale Veröffentlichungsnummer: WO 2022/043089

(56) Entgegenhaltungen:
- EP-A1- 0 982 303
- DE-A1- 1 793 310
- ABATE AGNESE ET AL: "Enzyme-Mediated Preparation of Chiral 1,3-Dioxane Odorants", HELVETICA CHIMICA ACTA, vol. 86, no. 3, 1 March 2003 (2003-03-01), pages 592 - 606, XP055800785, ISSN: 0018-019X, DOI: 10.1002/hlca.200390059
- BRUNS K ET AL: "Stereochemistry of cyclic compounds-I", TETRAHEDRON, ELSEVIER SIENCE PUBLISHERS, AMSTERDAM, NL, vol. 35, no. 21, 1 January 1979 (1979-01-01), pages 2523 - 2530, XP026614788, ISSN: 0040-4020, [retrieved on 19790101], DOI: 10.1016/0040-4020(79)88015-1
- FRITZ-LANGHALS E: "Separation of diastereomers by distillation - a new procedure for the synthesis of optically active heterocyclic carboxylic acids", ANGEWANTE CHEMIE INTERNATIONAL EDITION, VCH VERLAG, WEINHEIM, DE, vol. 32, no. 5, 1 May 1993 (1993-05-01), pages 753 - 754, XP002008344, ISSN: 0570-0833, DOI: 10.1002/ANIE.199307531
- ELIEL E L ET AL: "General Methods for the Separation of Diastereomers", STEREOCHEMISTRY OF ORGANIC COMPOUNDS, XX, XX, 1 January 1994 (1994-01-01), pages 374 - 381, XP002204163

## Beschreibung

### Gebiet der Erfindung

Die Erfindung betrifft ein destillatives Verfahren zur Herstellung einer diastereomerenangereicherten Riechstoffmischung mit blumiger Note umfassend Verbindungen der allgemeinen Formel (A): 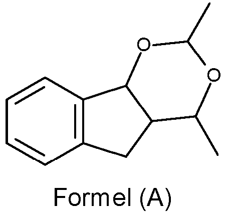 sowie die diastereomerenangereicherte Riechstoffmischung umfassend Verbindungen der allgemeinen Formel (A) herstellbar aus diesem Verfahren und ihre Verwendung als Riechstoff oder zur Herstellung einer Riechstoffzubereitung. Ferner betrifft die Erfindung die Verwendung der diastereomerenangereicherten Riechstoffmischung zum Vermitteln von, Modifizieren oder Verstärken einer blumigen Geruchsnote eines parfümierten Produktes oder zur Herstellung eines parfümierten Produktes selbst. Letztendlich betrifft die vorliegende Erfindung Riechstoffzubereitungen und parfümierte Produkte umfassend die diastereomerenangereicherte Riechstoffmischung.

### Stand der Technik

4,4a,5,9b-Tetrahydro-2,4-dimethylindeno[1,2-d][1,3]-dioxin ist ein beliebter Duft- oder Riechstoff, welcher in erster Linie einen transparenten, blumig-grünen Geruch aufweist und an den Duft von Magnolien, Geranien und Grapefruit (Pampelmuse) erinnert. Oftmals wird der Geruch dieser Verbindung auch als blumiggrün und sehr komplex beschrieben. Von daher findet Magnolan (Symrise AG), wie dieser Duft- oder Riechstoff kommerziell bezeichnet wird, insbesondere Anwendung, um blumige Duftnoten zu kreieren.

Weiterhin eignet sich Magnolan (Symrise AG) hervorragend zur Erzielung spezifischer blumiger Noten in floralen Kompositionen und zur Erzielung eines besonderen geruchlichen Effekts in Geruchskompositionen umfassend trockene und holzige Komponenten.

In der Offenlegungsschrift DE 1 793 310 werden 2,6-Dialkyl-4,5-indano-1,3-dioxanverbindungen, ein Verfahren zur Herstellung dieser Verbindungen sowie die Verwendung dieser Verbindungen in der Parfümherstellung und in Parfümwaren als Parfümgrundstoff beschrieben. Darin wird auch die Herstellung von 2,6-Dimethyl-4,5-(1',2'-indeno)-1,3-dioxan offenbart, welches einen rosenartigen Geruch ähnlich der Damascener Rose aufweist. Das hergestellte Produkt weist dabei zwei geometrische Isomere auf, welche in einem Verhältnis von 1,8 : 1 vorliegen. Insbesondere soll der besagt Riechstoff zur Ergänzung bzw. zum Ersatz von natürlichen Rosendüften Verwendung finden.

In WO 2010/142815 A2 ist ebenfalls eine Riechstoffmischung umfassend Magnolan offenbart. Die olfaktorischen Eigenschaften des Riechstoffs werden dabei als "weiße Blüte (insbesondere weiße Magnolie), blumig (rote Blüte, Pfingstrose, Geranie) und "indolartig" beschrieben.

Obwohl der bereits etablierte Riechstoff Magnolan (Symrise AG) grundsätzlich hervorragende olfaktorische Eigenschaften aufweist, ist allerdings oftmals beschrieben, dass dieser Riechstoff außerdem eine eher störende, technische und an Plastik erinnernde Geruchsnoten aufweist.

Wie in DE 1 793 310 beschrieben, handelt es sich in der Regel dabei um ein Produkt, in welchem die zueinander diastereomeren Isomere in nahezu gleicher Verteilung nebeneinander vorliegen.

### Aufgabe der Erfindung

Der Erfindung liegt demnach primär die allgemeine Aufgabe zugrunde, den kommerziell erhältlichen Riechstoff zu optimieren und die störenden Geruchsnoten zu eliminieren und somit einen intensiven, harmonischeren und "saubereren" blumigen Geruchseindruck zu gewährleisten und ein entsprechendes Verfahren zur Herstellung eines solchen optimierten Riechstoffs bereitzustellen.

Dabei ist ein schonendes Verfahren von höchster Wichtigkeit, um etwaige Zersetzungsprozesse und potenzielle Nebenreaktion sowie Wechselwirkungen zwischen derartigen Zersetzungsprodukten oder Nebenreaktionsprodukten zu minimieren, welche sich weiter nachteilig auf die olfaktorischen Eigenschaften des Produktes selbst oder der, den Reichstoff enthaltenden, Riechstoffzubereitungen und parfümierten Produkte auswirken könnten.

Zwar sind bereits Methoden bekannt, um Magnolan herzustellen, allerdings zeigen diese Verfahren in der Regel Nachteile in der Isomerenreinheit, insbesondere beim Upscaling, d.h. der Herstellung des Produktes in großem (industriellen) Maßstab: oftmals sind dabei auch nur geringe Ausbeuten und Selektivitäten, d.h. eine geringe Reinheit zu beobachten. Dies wirkt sich folglich nachteilig auf die Qualität des Produktes aus, und insbesondere auf die geruchlichen Eigenschaften wie zuvor erläutert.

Somit ist eine weitere Aufgabe der vorliegenden Erfindung, die Bereitstellung eines einfachen und schonenden Herstellungsverfahrens, welches es ermöglicht, einen optimierten blumigen Riechstoff in wenigen Schritten herzustellen und somit die gewünschten Produkte gleichzeitig kostengünstig und in hoher Ausbeute und Reinheit bereitzustellen.

Weiterhin betrifft eine Aufgabe, die Bereitstellung einer solchen Riechstoffmischung, ihre Verwendung als Riechstoff und zur Herstellung von Riechstoffzubereitungen und parfümierten Produkten sowie Riechstoffzubereitungen und parfümierten Produkten umfassend diese optimierte Riechstoffmischung.

Eine zusätzliche Aufgabe der Erfindung bezieht sich auf die Verminderung oder Maskierung eines unangenehmen Geruchs und/oder das Verstärken positiver Geruchseindrücke, vor allem das Verstärken von harmonischen, intensiven und "sauberen" blumigen Geruchseindrücken.

Eine weitere Aufgabe betrifft die schonende Anreicherung von Isomeren positiver Geruchseigenschaften und somit die gezielte Beeinflussung des Duftes, ohne die Duftstoffe zu schädigen bzw. geruchlich zu verändern.

Denkbar ist die Synthese beispielsweise ausgehend von dem Einsatz optisch aktiver Ausgangsstoffe, d.h. Ausgangsstoffen, bei denen die Stereoselektivität der Ausgangsverbindungen die Stereoisomerie der Produkte vorgibt, oder von isolierten Diastereomeren, wie zum Beispiel beschrieben in Abate A. et al., "Enzyme-Mediated Preparation of Chiral 1,3-Dioxane Odorants", HELVETICA CHIMICA ACTA, Bd. 86, Nr. 3, März 2003, Seiten 592-606. Allerdings ist ein derartiges Verfahren mit einer Vielzahl von aufwendigen und komplexen vorangehenden Synthese- und Aufreinigungsschritten zur Bereitstellung der isolierten Enantiomere oder Diastereomere der Ausgangsverbindungen verbunden. Dahingehend ergibt sich auch eine weitere Aufgabe der vorliegenden Erfindung, nämlich die Bereitstellung von diastereomerenangereicherten Magnolan-Produkten ohne die Notwendigkeit Isomerie-dirigierende Zwischenstufen als Ausgangs- oder Zwischenstufen bereitzustellen bzw. ein schnelles und effizientes Verfahren mit wenigen Verfahrensschritten bereitzustellen, welches gleichzeitig eine hohe Selektivität gewährleistet und zudem Zersetzungsreaktionen bzw. Nebenreaktionen weitestgehend unterbindet, die sich sonst nachteilig auf die Geruchseigenschaften auswirken könnten.

Die gestellten Aufgaben werden erfindungsgemäß durch die Gegenstände der unabhängigen Patentansprüche gelöst. Weitere Aspekte und bevorzugte Ausgestaltungen der vorliegenden Erfindung ergeben sich aus dem Wortlaut der abhängigen Patentansprüche, der nachfolgenden Beschreibung sowie den Ausführungsbeispielen.

### Zusammenfassung der Erfindung

Ein erster Gegenstand der vorliegenden Erfindung betrifft ein destillatives Verfahren zur Herstellung einer diastereomerenangereicherten Riechstoffmischung mit blumiger Note umfassend Verbindungen der allgemeinen Formel (A): 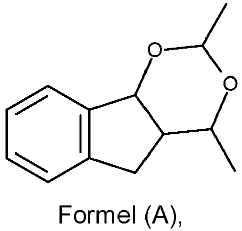 wobei die Riechstoffmischung die folgenden, zueinander diastereomeren, Enantiomerenpaare umfasst: 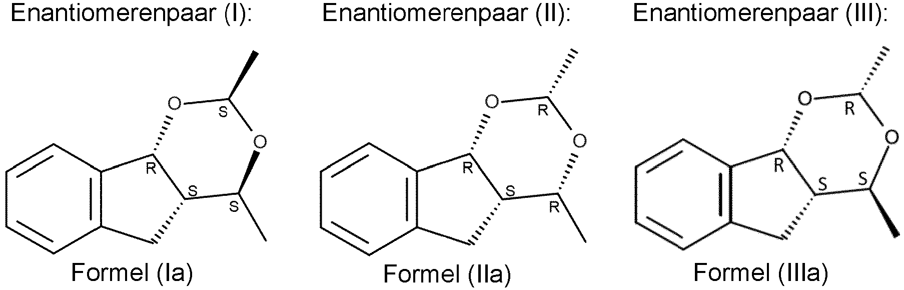 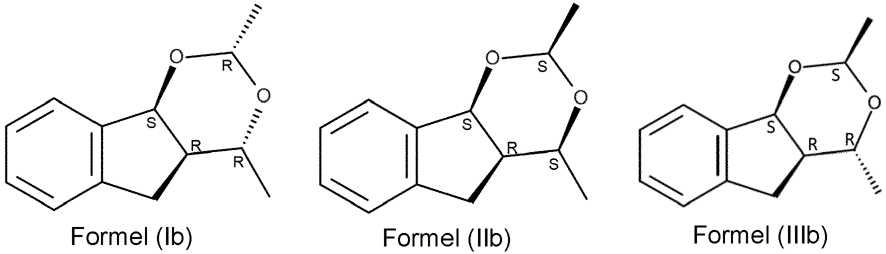 bei der das Mengenverhältnis des Enantiomerenpaars (I) und des Enantiomerenpaars (II) zueinander mindestens 10: ist und das Mengenverhältnis der Enantiomerenpaare (I) und (III) zueinander mindestens 50 : 1 ist, und wobei das Verfahren die folgenden destillativen Schritte umfasst:
(a) destillative Abtrennung eines Roh-Produkts umfassend Verbindungen der allgemeinen Formel (A) in einem ersten Destillationsschritt;
(b) anschließende Feindestillation des Roh-Produkts über einen oder mehrere Destillationsschritte unter Aufkonzentrierung des Enantiomerenpaars (I) im Verhältnis zu den Enantiomerenpaaren (II) und (III), wobei die Feindestillation mindestens 15 Trennstufen umfasst.

Es hat sich gezeigt, dass Riechstoffmischungen mit geringeren Mengen an Isomer (III) einen besseren Geruchseindruck des Magnolan Riechstoffs aufweisen. Weiterhin sollte die Riechstoffmischungen höhere Anteile an Isomer (I) im Verhältnis zu Isomer (II) enthalten. Diese selektive Anreicherung ist überraschend, da bisher keine selektiven Anreicherungsverfahren des Enantiomerenpaars (I) gegenüber den Enantiomerenpaaren (II) oder (III) bekannt waren, noch zu erwarten sind. Insbesondere derart hohe Anreicherungen sind überraschend. Weiterhin wurde diese Anreicherung mit verfahrenstechnisch vorteilhaften Prozessen erzielt, die gleichzeitig kostengünstig und effizient sind.

In einem zweiten Gegenstand umfasst die vorliegende Erfindung eine diastereomerenangereicherte Riechstoffmischung umfassend Verbindungen der allgemeinen Formel (A), wobei die Verbindungen der Formel (A) die zueinander diastereomeren, Enantiomerenpaare der Formeln (I), (II) und (III) umfasst und wobei das Mengenverhältnis des Enantiomerenpaars (I) und des Enantiomerenpaars (II) zueinander mindestens 10 : 1 ist und das Mengenverhältnis der Enantiomerenpaare (I) und (III) zueinander mindestens 501 ist (bezogen auf die gesamte Riechstoffmischung umfassend Verbindungen der allgemeinen Formel (A), d.h. die Verbindungen (I), (II) und (III)).

Dritter Gegenstand der vorliegenden Erfindung ist die Verwendung der diastereomerenangereicherten Riechstoffmischung als Riechstoff oder zur Herstellung einer Riechstoffzubereitung.

In einem vierten Aspekt betrifft die vorliegende Erfindung eine Riechstoffzubereitung enthaltend eine sensorisch wirksame Menge der diastereomerenangereicherten Riechstoffmischung.

Ein weiterer Gegenstand der vorliegenden Erfindung ist die Verwendung der diastereomerenangereicherten Riechstoffmischung in einer sensorisch wirksamen Menge zum Vermitteln, Modifizieren oder Verstärken einer blumigen Geruchsnote eines parfümierten Produktes oder zur Herstellung eines parfümierten Produktes als solches.

Letztendlich betrifft die vorliegende Erfindung in einem weiteren Aspekt ein parfümiertes Produkt, umfassend die diastereomerenangereicherte Riechstoffmischung oder eine Riechstoffzubereitung umfassend diese.

Überraschend wurde im Rahmen der vorliegenden Erfindung gefunden, dass sich mittels des hierin beschriebenen Verfahrens (vorzugsweise racemisch) diastereomerenangereicherte Riechstoffmischungen erhalten lassen, welche ein natürlicheres und intensives blumiges, rosiges, wärmeres, transparentes und strahlendes, nach Geranie duftendendes Geruchsprofil aufweisen, welches gegenüber kommerziell erhältlichem Magnolan als weniger technisch wahrgenommen wird und keine an Plastik erinnernde Geruchsnoten zeigt. Außerdem wird der Geruch gegenüber kommerziell erhältlichem Magnolan als weniger grün, intensiver rosig und weniger an Grapefruit erinnernd empfunden.

Weiterhin ist es möglich, mit dem hierin beschriebenen Verfahren, die (vorzugsweise racemisch) diastereomerenangereicherte Riechstoffmischung in sehr hoher Ausbeute und hervorragender Reinheit in nur wenigen Synthese- und Verfahrensschritten bereitzustellen, welche das hierin beschriebene Verfahren als ein höchst effizientes, hoch selektives und zuverlässiges, d.h. reproduzierbares, Verfahren auszeichnen. Die so erhaltenen Riechstoffe mit intensiv blumiger Note ermöglichen die Herstellung von verbesserten Riechstoffkompositionen mit besonderen geruchlichen Noten und Aspekten ohne die störenden technischen und nach Plastik riechenden Geruchskomponenten. Dementsprechend wird der Geruch der hierin beschriebenen Stoffe, Zubereitungen und Produkte als blumiger und "sauberer" empfunden.

Diese und weitere Aspekte, sowie weitere Merkmale und Vorteile der vorliegenden Erfindung werden für den Fachmann aus dem Studium der folgenden detaillierten Beschreibung und der Patentansprüche ersichtlich. Dabei kann jedes Merkmal aus einem Aspekt der Erfindung in einem anderen Aspekt der Erfindung eingesetzt oder ausgetauscht werden. Die in der vorliegenden Anmeldung enthaltenen Beispiele beschreiben die Erfindung, ohne diese einzuschränken.

Vorteilhafte Weiterbildungen und Varianten der Erfindung sind in den abhängigen Ansprüchen angegeben.

Alle Prozentangaben sind, sofern nicht anders angegeben, Gew.-%. Numerische Beispiele, die in der Form "von x bis y" angegeben sind, schließen die genannten Werte ein. Wenn mehrere bevorzugte numerische Bereiche in diesem Format angegeben sind, sind alle Bereiche, die durch die Kombination der verschiedenen Endpunkte entstehen, ebenfalls erfasst.

### Figuren

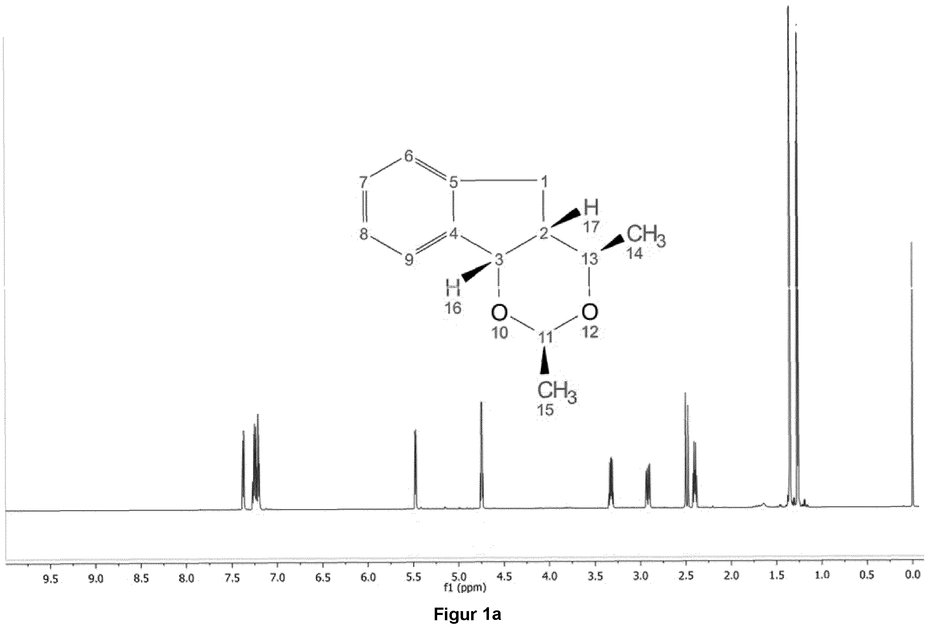

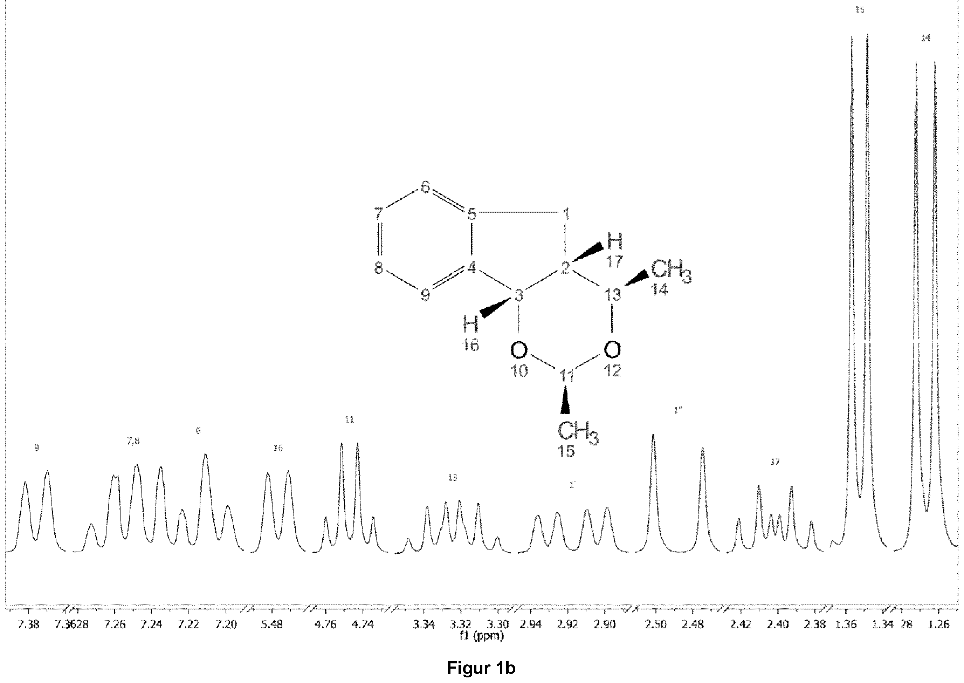

Figur 1a and 1b zeigen das¹H-NMR-Spektrum (600 MHz, Chloroform-d) einer ersten erfindungsgemäßen diastereomerenangereicherten Riechstoffmischung.

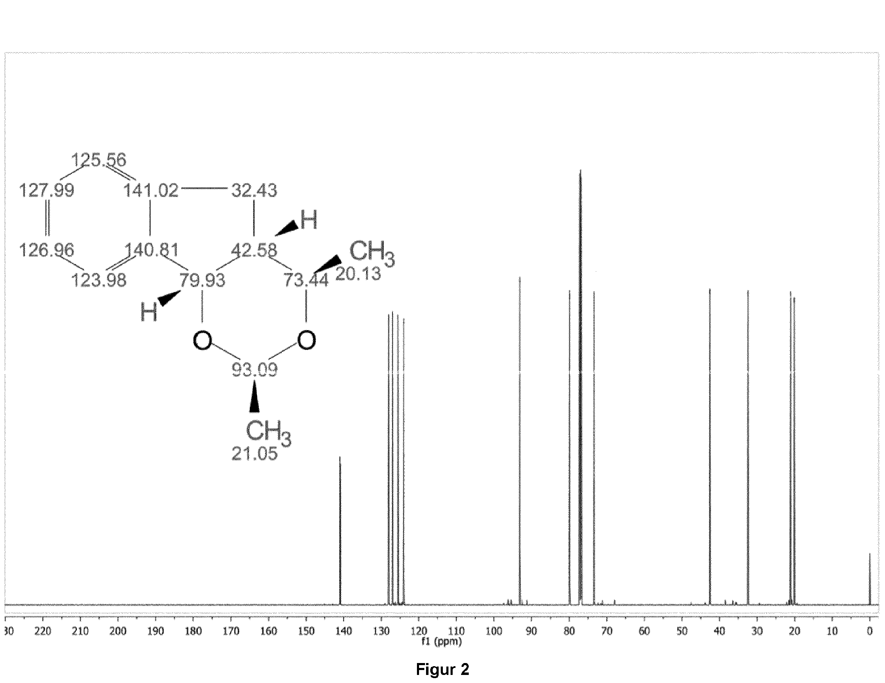

Figur 2 zeigt das ¹³C-NMR-Spektrum (151 MHz, CDCl₃) einer ersten erfindungsgemäßen diastereomerenangereicherten Riechstoffmischung.
Figur 3 zeigt die gaschromatographische Analyse einer zweiten erfindungsgemäßen diastereomerenangereicherten Riechstoffmischung.

### Detaillierte Beschreibung der Erfindung

In einem ersten Aspekt betrifft die vorliegende Erfindung ein destillatives Verfahren zur Herstellung einer (vorzugsweise racemisch) diastereomerenangereicherten Riechstoffmischung mit blumiger Note umfassend Verbindungen der allgemeinen Formel (A): 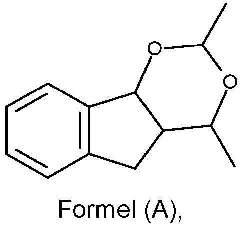 wobei die Riechstoffmischung die folgenden, zueinander diastereomeren, Enantiomerenpaare umfasst: 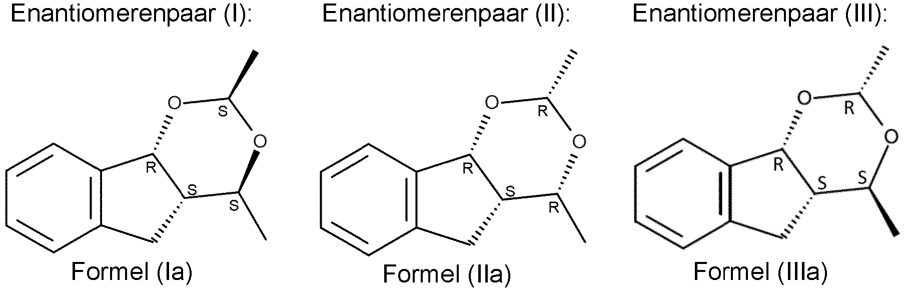 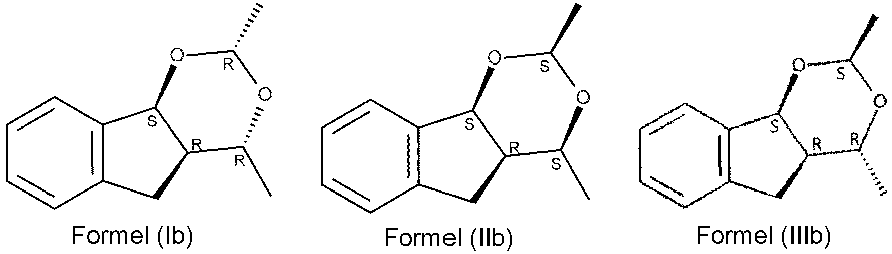 bei der das Mengenverhältnis des Enantiomerenpaars (I) und des Enantiomerenpaars (II) zueinander mindestens 10: ist und das Mengenverhältnis der Enantiomerenpaare (I) und (III) zueinander mindestens 50 : 1 ist, und wobei das Verfahren die folgenden destillativen Schritte umfasst:
(a) destillative Abtrennung eines Roh-Produkts umfassend Verbindungen der allgemeinen Formel (A) in einem ersten Destillationsschritt;
(b) anschließende Feindestillation des Roh-Produkts über einen oder mehrere Destillationsschritte unter Aufkonzentrierung des Enantiomerenpaars (I), insbesondere im Verhältnis zu den Enantiomerenpaaren (II) und (III), wobei die Feindestillation mindestens 15 Trennstufen umfasst.

Dem Fachmann ist bewusst, dass die Verbindung der allgemeinen Formel (A) eine chirale Verbindung ist. Weiterhin ist dem Fachmann geläufig, dass diese Enantiomerenpaare zueinander diastereomer sind, während die Enantiomere des Enantiomerenpaars, wie die Bezeichnung bereits andeutet, untereinander enantiomer sind, d.h. sie verhalten sich wie Bild und Spiegelbild. Eine Anreicherung eines Diastereomers bedeutet demzufolge die Erzeugung eines mengenmäßigen Überschusses eines dieser Enantiomerenpaare, hier des Enantiomerenpaars (I), relativ zu den anderen vorhandenen Enantiomerenpaaren der Riechstoffmischung, hier den Enantiomerenpaaren (II) und (III) der allgemeinen Formel (A).

Der Begriff "diastereomerenangereichert" bezeichnet demnach in diesem Zusammenhang den Anteil eines Diastereomers, d.h. eines bevorzugten Enantiomerenpaars, im Gemisch mit den anderen möglichen Diastereomeren der betrachteten Verbindung, d.h. der allgemeinen Formel (A). Unter dem Begriff "diastereomerenangereichert" ist dabei zu verstehen, dass die im Rahmen des hierin beschriebenen Verfahrens erhältlichen Riechstoffmischungen einen deutlich höheren Gehalt des Enantiomerenpaars (I) relativ zu den dazu diastereomeren Enantiomerenpaaren (II) und (III) aufweisen, als die mittels herkömmlicher Verfahren erhältlichen und kommerziell vertriebenen Magnolan-Produkte. Folglich beschreibt der Begriff "diastereomerenangereichert" im Rahmen der Erfindung das Vorliegen eines Diastereomers, d.h. Enantiomerenpaars (vorzugsweise des Enantiomerenpaars (I)), wie zuvor definiert, mit einem Anteil im Gemisch mit den anderen möglichen diastereomeren Isomeren in einem Bereich von > 50 Gew.-% und 100 Gew.-%. Insbesondere ist unter dem Begriff "diastereomerenangereicherte Riechstoffmischung" eine solche zu verstehen, welche zu mindestens 80 Gew.-% bis 99,9 Gew.-%, bevorzugt 90 Gew.-% bis 99,8 Gew.-% und besonders bevorzugt 95 Gew.-% bis 99,5 Gew.-% des Enantiomerenpaars (I), umfassend die Enantiomere der Formeln (la) und (Ib), neben zusammen bis zu 20 Gew.-%, bevorzugt bis zu 10 Gew.-%, und besonders bevorzugt 5 Gew.-% bis 0,5 Gew.-% der weiteren dazu diastereomeren Enantiomerenpaare (II) und/oder (III) enthält.

Im Kontext der vorliegenden Erfindung werden unter der Bezeichnung "Verbindungen der Formel (I)" oder "Enantiomerenpaar der Formel (I)" oder "Enantiomerenpaar (I)", "Verbindung (I)" sowie "Isomer (I)" sowohl die einzelnen enantiomeren Verbindungen der Formel (I) und folglich die Enantiomere (la) und (Ib) als auch sämtliche Mischungen dieser Enantiomere in jedem beliebigen Mischungsverhältnis verstanden. Das heißt, Ausführungen in der nachfolgenden Beschreibung betreffend das "Enantiomerenpaar (I)" gelten sowohl für eine einzelne Verbindung der Formel (I) und somit die Enantiomere (la) und (Ib) als auch für Mischungen, bestehend aus oder umfassend die Enantiomere (la) und (Ib) in jedem Mischungsverhältnis. Gleiches gilt für die Bezeichnungen "Enantiomerenpaar (II)" und "Enantiomerenpaar (III)".

Dabei sind jedoch die Enantiomere (la) und (Ib), (IIa) und (IIb) sowie (IIIa) und (IIIb) vorzugsweise jeweils zueinander derart umfasst, sodass jeweils die Enantiomerenpaare (I), (II) und (III) in einer racemischen Mischung der jeweiligen Enantiomere (la) und (Ib), (IIa) und (IIb) bzw. (IIIa) und (IIIb) vorliegen. Folglich betrifft eine weitere bevorzugte Ausführungsform die Bereitstellung einer racemisch diastereomerenangereicherten Riechstoffmischung, worin die Enantiomerenpaare (I), (II) und (III) jeweils in racemischer Form vorliegen, insbesondere jedoch das Enantiomerenpaar (I). Derartige racemische Verbindungen und folglich racemisch diastereomerenangereicherten Riechstoffmischungen zeigen einen besonders ausgewogenen und harmonischen Geruch.

Die Verbindungen der allgemeinen Formel (A) tragen die folgenden Bezeichnungen:
Enantiomerenpaar (I):
   Enantiomer (la):
      (2S,4S,4aS,9bR)-2,4-dimethyl-4,4a,5,9b-tetrahydroindeno[1,2-d][1,3]dioxine
   Enantiomer (Ib):
      (2R,4R,4aR,9bS)-2,4-dimethyl-4,4a,5,9b-tetrahydroindeno[1,2-d][1,3]dioxine
Enantiomerenpaar (II):
   Enantiomer (IIa):
      (2R,4R,4aS,9bR)-2,4-dimethyl-4,4a,5,9b-tetrahydroindeno[1,2-d][1,3]dioxine
   Enantiomer (IIb):
      (2S,4S,4aR,9bS)-2,4-dimethyl-4,4a,5,9b-tetrahydroindeno[1,2-d][1,3]dioxine
Enantiomerenpaar (III):
   Enantiomer (IIIa):
      (2R,4S,4aS,9bR)-2,4-dimethyl-4,4a,5,9b-tetrahydroindeno[1,2-d][1,3]dioxine
   Enantiomer (IIIb):
      (2S,4R,4aR,9bS)-2,4-dimethyl-4,4a,5,9b-tetrahydroindeno[1,2-d][1,3]dioxine

Die resultierende (vorzugsweise racemisch) diastereomerenangereicherte Riechstoffmischung, umfassend die Verbindungen der allgemeinen Formel (I), umfasst dabei das Enantiomerenpaar (I) relativ zum Enantiomerenpaar (II) in der Mischung in einem Verhältnis von mindestens 10:1. Vorzugsweise, ist das Mengenverhältnis des Enantiomerenpaars (I) und des Enantiomerenpaars (II) in der Mischung zueinander mindestens 15:1, bevorzugter mindestens 20: 1, noch bevorzugter mindestens 50: 1. Weiter bevorzugt ist das Mengenverhältnis dieser diastereomeren Enantiomerenpaare zueinander mindestens 100 : 1.

Wie in DE 1 793 310 beschrieben, umfasst das 2,6-Dialkyl-4,5-indano-1,3-dioxan zwei geometrische Hauptisomere, welche zueinander lediglich in einem Verhältnis von 1,8 : 1 vorliegen. Für sich genommen ist die Prins-Reaktion grundsätzlich als ein sehr unselektives Verfahren anzusehen, welches in der Regel zur Bildung der Enantiomerenpaare in nahezu gleichen Mengen führt.

Im Rahmen der vorliegenden Erfindung hat sich überraschend gezeigt, dass allerdings eines dieser Isomere, welche in der herkömmlichen Magnolan-Synthese normalerweise in nahezu statistischer Verteilung anfallen, parfümistisch zu bevorzugen ist. Durch das hierin beschriebene Verfahren ist es möglich, das bevorzugte Isomer, Isomer (I), in der anfallenden Riechstoffmischung deutlich gegenüber dem zweiten Hauptisomer anzureichern und so die geruchlichen Eigenschaften des resultierenden Riechstoffs positiv zu beeinflussen.

So war es möglich, mittels des vorliegenden Verfahrens den Anteil des bevorzugten Isomers in der Reichstoffmischung auf über 95 Gew.-% bezogen auf die anderen enthaltenen Isomere anzureichern und somit die positiven Geruchskomponenten der Riechstoffmischung deutlich hervorzuheben. Es hat sich ferner überraschend gezeigt, dass insbesondere die Verbindungen des Enantiomerenpaars (I) für die positiven Geruchszüge der Riechstoffmischung verantwortlich sind und die oben beschriebene Isomerenverteilung die Erzeugung eines besonders intensiv blumigen, "sauberen" und balancierten Geruchs ermöglicht, während das hierin beschriebene Verfahren als solches hoch selektiv, effizient und schonend ist.

Eine weitere Ausgestaltung des hierin beschriebenen Verfahrens betrifft somit die Bereitstellung einer diastereomerenangereicherten Riechstoffmischung, wie oben beschrieben, worin der Anteil des Enantiomerenpaars (I) in der Riechstoffmischung mindestens 95 Gew.-% beträgt und noch bevorzugter mindestens 97,5 Gew.-%, noch bevorzugter mindestens 98,5 Gew.-% bezogen auf die Summe der Enantiomerenpaare (I), (II) und (III). Eine derartige Riechstoffmischung weist ein überaus angenehmes und intensiv blumiges Geruchsprofil auf, worin keine technischen oder sonst in irgendeiner Form als negativ wahrgenommenen Geruchsnoten zu erkennen sind, d.h. ein "sauberes" intensiv blumiges Geruchsprofil.

Dementsprechend betrifft eine weitere Ausgestaltung des vorliegenden Verfahrens zur Herstellung die Bereitstellung einer diastereomerenangereicherte Riechstoffmischung, worin mindestens 95 Gew.-% des (vorzugsweise racemischen) Enantiomerenpaars (I) umfasst sind, vorzugsweise 97,5 Gew.-%, noch bevorzugter mindestens 98,5 Gew.-% bezogen auf die Summe der Enantiomerenpaare (I), (II) und **(III).**

Derartig reine bzw. stark angereicherte diastereomerenangereicherte Riechstoffmischungen des Magnolan-Typs sind bis zu dem heutigen Zeitpunkt nicht bekannt. Weiterhin ist zu betonen, dass gefunden wurde, dass eine derartige Riechstoffmischung einen besonders ausgewogenen und besonders natürlichen warmen und blumigen Geruch aufweist. Ferner ist zu betonen, dass das hierin beschriebene Verfahren ein hoch selektives Verfahren beschreibt, welches gleichzeitig besonders schonend ist.

Ferner wurde überraschend gefunden, dass insbesondere racemische Enantiomerenpaare, insbesondere racemische Enantiomerenpaare der Formel (I), entscheidend zu einem ausgewogenen, natürlichen, blumigen und intensiven Geruchsprofil beitragen. So konnte beobachtet werden, dass diastereomerenangereicherte Riechstoffmischung, wie hierin beschrieben, umfassend die Verbindungen der allgemeinen Formel (A) und insbesondere höhere Anteile des racemischen Enantiomerenpaars (I) gegenüber den Enantiomerenpaaren (II) und/oder (III), zu diesem außergewöhnlichen Geruchsprofil beitragen: So wurde gefunden, dass das resultierende Produkt einen positiven und optimierten, natürlichen, intensiv blumigen, rosigen, transparenten, etwas wärmeren, strahlenden und nach Geranium riechenden Geruch aufweist, und keine technischen und an Plastik erinnernden Nuancen zeigt. Insbesondere die ausgewogenen, intensiven, außergewöhnlich natürlichen und "sauberen" blumigen Geruchsnuancen zeichnen die vorliegenden Riechstoffmischung aus, welche sich auf die Diastereomerenanreicherung mit dem vorzugsweise racemischen Enantiomerenpaar (I) in der Riechstoffmischung des ersten Aspekts zurückführen lassen und die Eignung der vorliegenden Riechstoffmischung als Riechstoff bzw. Riechstoffbasis in einer Vielzahl von komplexen Riechstoffzubereitungen und parfümierten Produkten begründen.

Außerdem umfasst die so erhaltene Riechstoffmischung, umfassend die Verbindungen der allgemeinen Formel (A), die Enantiomerenpaare (I) und (III) zueinander vorzugsweise in einem Mengenverhältnis von mindestens 50 : 1. Weiter bevorzugt ist das Mengenverhältnis der Enantiomerenpaare (I) und (III) zueinander mindestens 100 : 1; noch bevorzugter mindestens 1000 : 1 und am bevorzugtesten ist das entsprechende Mengenverhältnis mindestens 1360 : 1.

Somit war es gleichzeitig möglich, die Riechstoffkomponente, welche primär für den technischen und nach Plastik riechenden Geruchseindruck verantwortlich war, deutlich zu minimieren und folglich die Riechstoffmischung entsprechend zu optimieren.

Entsprechend weist die vorliegende Riechstoffmischung vorzugsweise einen deutlich höheren Anteil der Enantiomere (la) und (Ib) des Enantiomerenpaars (I) relativ zu den anderen Diastereomeren in der Riechstoffmischung auf, d.h. den Enantiomeren (IIa) und (IIb) sowie (IIIa) und (IIIb) der Enantiomerenpaare (II) und (III).

Dabei wurde im Rahmen der vorliegenden Erfindung überraschend gefunden, dass sich folglich mittels des hierin beschriebenen Verfahrens an Enantiomerenpaar (I) (vorzugsweise racemisch) diastereomerenangereicherte Riechstoffmischungen erhalten lassen, welche ein intensiv und natürlich blumiges, rosiges, im Allgemeinen etwas wärmeres, transparentes und strahlendes, nach Geranie duftendendes Geruchsprofil aufweisen und gegenüber kommerziell erhältlichen Magnolan als weniger technisch wahrgenommen werden und keine an Plastik erinnernde Geruchsnoten zeigen (und somit als "sauberer" wahrgenommen werden). Folglich ist der Geruch gegenüber kommerziell erhältlichem Magnolan intensiver, natürlicher, balancierter, blumiger und "sauberer".

Die Verfahrensschritte (a) und (b) des destillativen Verfahrens zur Herstellung einer (vorzugsweise racemisch) diastereomerenangereicherten Riechstoffmischung werden im Folgenden näher erläutert.

Ein erster Schritt (a) des hierin beschriebenen destillativen Verfahrens betrifft die destillative Abtrennung eines Roh-Produkts umfassend Verbindungen der allgemeinen Formel (A). Dabei handelt es sich bei dem Produkt, welches diesem ersten Destillationsschritt unterworfen wird, um ein Magnolan-Roh-Produkt, welches vorzugsweise aus der Synthese ausgehend von Inden und Paraldehyd gewonnen wurde. Die Bereitstellung eines solchen Produktes wird weiter unten beschrieben. Im Allgemeinen handelt es sich dabei jedoch um ein Magnolan-Produkt, welches keinem zusätzlichen aufreinigenden Schritt unterzogen wurde und welches direkt im Anschluss an die Umsetzung der Edukte im Reaktionsgemisch als solches vorliegt.

Primär dient dieser erste Destillationsschritt der Grob-Destillation um etwaige Lösungsmittelreste, Katalysator-Rückstände und nicht umgesetzte Ausgangsverbindungen bzw. Edukte von der, im Wesentlichen noch nicht diastereomerenangereicherten Riechstoffmischung umfassend die Verbindungen der Formel (A) bzw. die Enantiomerenpaare (I), (II) und (III), herauszutrennen. Gegebenenfalls wird dieser Verfahrensschritt, falls nötig, auch unter vermindertem Druck durchgeführt.

Basierend auf diesem ersten Destillationsschritt des Verfahrens ist es somit möglich, die Riechstoffmischung zunächst durch Abtrennung eines Magnolan-Roh-Produktes in ausreichender chemischer Reinheit, in Form eines Roh-Produktes bereitzustellen, und chemische Wechselwirkungen mit anderen, in dem Roh-Produkt enthaltenen Substanzen zu vermeiden, welche sich negativ auf den olfaktorischen Gesamteindruck der finalen diastereomerenangereicherten Riechstoffmischung auswirken könnten.

Das Roh-Produkt umfassend Verbindungen der allgemeinen Formel (A) weist beispielsweise im Anschluss an die Grob-Destillation eine chemische Reinheit von mindestens 80% auf, vorzugsweise von mindestens 90%.

Im Anschluss an diesen ersten Destillationsschritt umfasst das hierin beschriebene Verfahren zur Herstellung einer diastereomerenangereicherten Riechstoffmischung einen weiteren Destillationsschritt, die Feindestillation des Roh-Produkts umfassend Verbindungen der allgemeinen Formel (A). Diese Feindestillation erfolgt dabei über einen oder mehrere Destillationsschritte unter Aufkonzentrierung des Enantiomerenpaars (I), wobei die Feindestillation mindestens 15 Trennstufen umfasst. Vorzugsweise umfasst die Feindestillation jedoch mindestens 18 Trennstufen und am bevorzugtesten mindestens 20 Trennstufen.

Das durch gewöhnliche Synthese erhältliche Magnolan der Formel (A) weist für gewöhnlich eine Zusammensetzung auf, in welcher die Isomere (I) und (II) zueinander nahezu statistisch verteilt sind, maximal jedoch in einem Verhältnis von 2 : 1.

Das erfindungsgemäß nach den Schritten (a) und (b) durchzuführende destillative Verfahren ermöglicht demnach auch die Herstellung von diastereomerenangereicherten Riechstoffmischungen durch gezielte Feindestillation aus einer im Wesentlichen nicht-diastereomerenangereicherten Ausgangsverbindung, welche im Grunde weder einen nennenswerten Enantiomerennoch einen nennenswerten Diastereomerenüberschuss eines oder mehrerer der darin enthaltenen geometrischen Isomere aufweist.

Vorzugsweise wird der Vorlauf der Grob-Destillation der anschließenden Feindestillation unterworfen. So lassen sich noch höhere (diastereomeren) Reinheiten und Ausbeuten erzielen.

Ferner eignet sich das hierin beschriebene Verfahren hervorragend für die Bereitstellung der Riechstoffmischung in großem Maßstab und ermöglicht es somit, den Bedarf der Parfümindustrie vollständig zu decken. Insbesondre von Bedeutung ist dabei die gleichbleibende Qualität des Produktes von Charge zu Charge, welche mit dem vorliegenden Verfahren gewährleistet werden kann. Das Verfahren zeigt dabei eine besonders hohe Selektivität, macht nur wenige Verfahrensschritte notwendig und ist besonders schonend, sodass etwaige Zersetzungs- oder Nebenreaktionen effektiv verhindert werden können. Folglich zeichnet sich das hierin beschriebene Verfahren als ein besonders effizientes Verfahren aus.

Insbesondere sind somit Anreicherungen des Enantiomerenpaars (I) mit einem Anteil von über 95 Gew.-% bezogen auf die Summe der Enantiomerenpaare (I), (II) und (III) in der anfallenden Riechstoffmischung möglich.

Diese Anreicherung ist überraschend, da bisher keine selektiven Anreicherungen von dem Enantiomerenpaar (I) gegenüber den Enantiomerenpaaren (II) oder (III) bekannt waren, noch zu erwarten sind.

In einer weiteren bevorzugten Ausführungsform betrifft die vorliegende Erfindung ein Verfahren zur Herstellung einer diastereomerenangereicherten Riechstoffmischung wie zuvor beschrieben, welches vor den destillativen Schritten (Grob- und Feindestillation) weiterhin umfasst:
- die Bereitstellung von Paraldehyd der allgemeinen Formel (IV) oder Acetaldehyd, 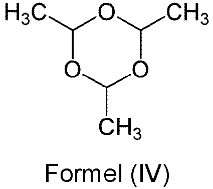 sowie
- dessen Umsetzung mit Inden der allgemeinen Formel (V) unter saurer Katalyse im Lösungsmittel, 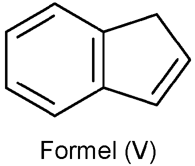 wobei die Umsetzung des Paraldehyds oder Acetaldehyds mit Inden bei Temperaturen unterhalb von 10 °C erfolgt; und
- Gewinnung des Roh-Produkts umfassend Verbindungen der allgemeinen Formel (A).

Folglich beschreibt diese bevorzugte Ausführungsform die Synthese des Magnolan-Roh-Produktes, welches dem destillativen Verfahren des ersten Aspekts unterworfen werden kann und vorzugsweis welches dem destillativen Verfahren des ersten Aspekts auch unterworfen wird.

In einer weiteren Ausgestaltung dieser Synthese, erfolgt die Umsetzung der Verbindungen der allgemeinen Formeln (IV) und (V) unter saurer Katalyse, vorzugsweise in Gegenwart von verdünnter Schwefelsäure in Toluol.

Im Wesentlichen entspricht der Mechanismus der Synthese den Grundzügen der sogenannten klassischen Prins-Reaktion, d.h. einer säurekatalysierten Carbonyl-En-Reaktion, einer Form der Cycloadditionen, welche die elektrophile Addition eines Aldehyds oder Ketons an ein Alken oder Alkin beschreibt.

Folglich betrifft das hierin beschriebene Verfahren weiterhin die Bereitstellung des Roh-Produktes (2,4,4a,9b)-2,4-dimethyl-(4,4a,5,9b)-tetrahydronindeno[1,2d][1,3]dioxin mittels klassischer Prins-Reaktion unter optimierten Verfahrensbedingungen.

Die Umsetzung der Verbindungen der Formeln (IV) und (V) des erfindungsgemäßen Verfahrens gemäß dem ersten Aspekt zum Roh-Produkts der allgemeinen Formel (A) wird dabei bevorzugt über eine Dauer von etwa 5 Stunden durchgeführt. Weiterhin ist es bevorzugt, dass die saure Katalyse durch Umsetzung in einer Emulsion aus verdünnter Schwefelsäure und Toluol durchgeführt wird.

Mit dieser Methode konnten bezüglich dem (noch nicht diastereomerenangereicherten) Roh-Produkt bereits sehr hohe chemische Reinheiten und Ausbeuten erzielt werden.

Vorzugsweise sind an der Umsetzung keine weiteren Substanzen, wie beispielsweise Antioxidantien, Iod, Enzyme oder weitere Salze beteiligt.

Dieser Reaktionsschritt der Edukte weist dabei als solcher keinerlei Selektivität hinsichtlich der einzelnen enantiomeren bzw. diastereomeren Isomere der Verbindungen der allgemeinen Formel (A) auf. In der Regel liegt folglich das Roh-Produkt der Formel (A) als Gemisch einer, im Wesentlichen, statistisch verteilten Menge der einzelnen geometrischen Isomere vor, d.h. die Enantiomerenpaare (I), (II) und (III) liegen nahezu statistisch verteilt im Roh-Produkt der allgemeinen Formel (A) vor.

Die dabei hauptsächlich gebildeten Enantiomere und (vorzugsweise racemischen) Diastereomere lassen sich dabei mit den Formeln (la), (Ib), (IIa), (IIb), (IIIa) und (IIIb) bzw. (I), (II) und (III) beschreiben.

Dabei wurde gefunden, dass die zueinander diastereomeren Enantiomerenpaare (I), (II) und (III) voneinander verschiedene Geruchsprofile aufweisen. So kann der Geruch des Enantiomerenpaars (I) als blumig, rosig, transparent, strahlend und nach Geranium riechend beschrieben werden, während das Enantiomerenpaar (II) einen blumigen, grünen, etwas technischen, nach Grapefruit riechenden und im Vergleich zum Enantiomerenpaar (I) schwächeren Geruchseindruck erweckt. Ferner wurde überraschend gefunden, dass das Enantiomerenpaar (III) ein blumiges, technisches, unsauberes und nach Plastik riechendes Geruchsprofil aufweist. Eine Geruchsbeschreibung dieses Enantiomerenpaars (III) konnte nun erstmals im Rahmen der vorliegenden Erfindung identifiziert und beschrieben werden.

Ferner hat sich gezeigt, dass das Isomer (I) das geruchlich wertvollste Isomer der drei identifizierten zueinander diastereomeren Isomere ist, sodass die diastereomerenselektive Anreicherung der Riechstoffmischung mit den geometrischen Isomeren des vorzugsweise racemischen Enantiomerenpaars (I) besonders erstrebenswert ist und Bedarf an einem effizienten und selektivem Verfahren besteht, um insbesondere die Isomere des Enantiomerenpaars (I) in der herzustellenden Riechstoffmischung anzureichern.

Gleichzeitig ist somit eine Reduktion des Anteils des Enantiomerenpaars (III) hinsichtlich der als negative empfundenen Geruchscharakteristika bevorzugt. Dabei ist es von besonderem Interesse, dass das Mengenverhältnis der Enantiomerenpaare (I) und (III) zueinander mindestens 50 : 1 ist.

Gleichzeitig, bzw. unabhängig davon ist es ferner erstrebenswert, dass das Mengenverhältnis des Enantiomerenpaars (I) und des Enantiomerenpaars (II) zueinander mindestens 10 : 1 ist.

Es wurde überraschend gefunden, dass, wenn die diastereomeren Enantiomerenpaare (I), (II) und (III) in diesen Verhältnissen zueinander vorliegen, besonders intensive strahlende, wärmere, natürlichere, und transparente florale bzw. blumige Geruchsprofile für die entsprechenden Riechstoffmischungen erzielt werden können, welche eine besondere Balance aufweisen.

Standardmäßige Verfahren zur Isolierung eines Enantiomers oder Diastereomers bzw. Enantiomerenpaars sind allerding in der Regel mit einer Vielzahl von Zwischenschritten verbunden, wie beispielsweise der gezielten Bereitstellung von bestimmten Edukten in vorgegeben Stereoisomerien oder die enzymatische Steuerung der Geometrie. Weiterhin sind solche Verfahren in der Regel weder schonend noch in ihrer Gesamtheit besonders effizient oder selektiv.

Mit dem hierin beschrieben Verfahren zur Bereitstellung einer (vorzugsweise racemisch) diastereomerenangereicherten Riechstoffmischung umfassend die Verbindungen der allgemeinen Formel (A) und die Enantiomerenpaare (I), (II) und (III) ist es allerdings gelungen, den Anteil des Enantiomerenpaars (I) relativ zu dessen Diastereomeren derart anzureichern, sodass das resultierende Produkt einen positiven und optimierten, natürlichen, intensiv blumigen, rosigen, transparenten, etwas wärmeren, strahlenden und nach Geranium riechenden Geruch verströmt. Insbesondere die hohe Intensität, Sauberkeit und Natürlichkeit des Geruchs zeichnen die vorliegenden Riechstoffmischung als hervorragenden Riechstoff/Riechstoffmischung bzw. als ausgezeichnete Riechstoffbasis für die weitere Verwendung in der Herstellung von natürlich und intensiv blumig riechenden Riechstoffzubereitungen und parfümierten Produkten aus. In Zusammenhang mit dem verminderten technischen, unsauberen und nach Plastik riechenden Nuancen ergibt sich so eine exzellente Riechstoffmischung mit einem außergewöhnlichen, ausgewogenen, intensiven und "sauberen" blumigen Duft, welche sich hervorragend in komplexe Duftkreation einarbeiten lässt. Diese Geruchsnuancen lassen sich mit Riechstoffmischungen erzielen, welche die Enantiomerenpaare (I), (II) und (III) in den erfindungsgemäßen Verhältnissen aufweisen. Ein gewisser Anteil von Enantiomerenpaar (II) in der Riechstoffmischung kann auch zu einer intensiver blumigeren, grüneren und somit natürlicher blumigen Komponente beitragen. Besonders natürliche Nuancen sind in der Parfümindustrie von besonderem Interesse. Somit kann mit der vorliegend beschriebenen Riechstoffmischung der Parfümindustrie eine Duftkomponente zur Verfügung gestellt werden, welche sich aufgrund des besonders intensiv-blumigen, ausgewogenen und überraschend "sauberen" Geruchsprofils, besonders hervorragend als natürlich-blumige Geruchskomponente in diversen Zubereitungen und Produkten eignet.

Weiterhin konnte nun ein hoch selektives, einfaches und schonendes Herstellungsverfahren entwickelt werden, welches ermöglicht, einen optimierten intensiv blumigen Riechstoff in wenigen Schritten herzustellen und somit diesen gleichzeitig kostengünstig, äußerst rein und in hoher Ausbeute bereitzustellen.

Gleichzeitig ermöglicht das hierin beschriebene Verfahren die effiziente und hoch selektive Diastereomerenanreicherung des Enantiomerenpaars (I) ausgehend von der Synthese des Roh-Produktes mittels klassischer Prins-Reaktion in hohen Ausbeuten und in hoher Produkt-Reinheit.

Ferner wurde gefunden, dass sich mit dem hierin beschriebenen Verfahren Riechstoffmischungen herstellen lassen, welche einen blumigen, rosigen, transparenten, strahlenden und an Geranium erinnernden Geruch aufweisen. Die so hergestellten diastereomerenangereicherten Riechstoffmischungen zeigen dabei keine technischen und an Plastik erinnernden Geruchsnoten. Auch tritt der Geruch nach Grapefruit deutlich in den Hintergrund, sodass die so hergestellte Riechstoffmischung im Wesentlichen einen deutlich strahlenderen, intensiveren und natürlicher blumigen Geruchseindruck als kommerziell erhältliche Magnolan-Produkte aufweist.

In einer weiteren bevorzugten Alternative wird das hierin beschrieben Verfahren zur Herstellung einer diastereomerenangereicherten Riechstoffmischung nach dem ersten Aspekt, derart durchgeführt, dass die Feindestillation des destillativen Verfahrens in einem kontinuierlichen Prozess erfolgt.

Dieser kontinuierliche Verfahrensprozess ermöglicht es, die Anzahl von Zwischenschritten, wie beispielsweise das Wiederbefüllen oder Abkühlen und Aufheizen der Apparatur, zu reduzieren und ist daher im Vergleich zu sogenannten Chargenprozessen wirtschaftlicher und gewährleistet eine konstante, d.h. gleichbleibende Produktqualität. Dementsprechend eignet sich das vorliegende Verfahren auch für Herstellungen der beschrieben Riechstoffmischung im industriellen Maßstab und erlaubt so, die hochwertige Herstellung der diastereomerenangereicherten Riechstoffmischung in Mengen von über 180 kg mit ausgezeichneten Dufteigenschaften und hervorragender Reinheit.

In einer weiteren bevorzugten Ausgestaltung wird der Schritt der Feindestillation bei dem erfindungsgemäßen destillativen Verfahren gemäß dem ersten Aspekt zur Herstellung einer diastereomerenangereicherten Riechstoffmischung bei einem Rücklaufverhältnis von mindestens 5 : 1 durchgeführt. Vorzugsweise beträgt das besagte Rücklaufverhältnis mindestens 7 : 1 und am bevorzugtesten mindestens 10:1, um besonders hohe Anteile des Enantiomerenpaars (I) relativ zu den Enantiomerenpaaren (II) und (III) zu erreichen und die als negativ und technisch beschrieben bzw. nach Plastik riechenden Geruchskomponenten zu minimieren, während die intensiv-blumigen und positiven Geruchseindrücke in den Vordergrund treten.

Im Rahmen der vorliegenden Erfindung wurde dabei überraschend gefunden, dass ein derartiges Rücklaufverhältnis in Kombination mit den zuvor beschriebenen Parametern zu einer besonders effizienten und starken Diastereomerenanreicherung des Enantiomerenpaars (I) in der hierin beschriebenen Riechstoffmischung führt.

Weiterhin bevorzugt wird in dem erfindungsgemäßen Verfahren gemäß dem ersten Aspekt die Feindestillation des destillativen Verfahrens bei Temperaturen zwischen 120 °C und 150 °C durchgeführt, vorzugsweise beträgt die Kopftemperatur allerdings zwischen 125 °C und 145 °C, noch bevorzugter zwischen 135 °C und 140 °C.

Dies ermöglicht eine effiziente und hoch selektive Anreicherung des bevorzugten Diastereomers, d.h. des Enantiomerenpaars (I) und gewährleistet die Reproduzierbarkeit des angestrebten Geruchsprofils.

In einer weiter bevorzugten Ausgestaltung wird die Feindestillation des hierin beschriebenen destillativen Verfahrens zur Herstellung einer diastereomerenangereicherten Riechstoffmischung bei einem verminderten Druck von etwa 1 mbar bis 100 mbar durchgeführt, vorzugsweise bei einem verminderten Druck von etwa 1 mbar bis 50 mbar, besonders bevorzugt bei einem verminderten Druck von etwa 1 mbar bis 10 mbar und noch bevorzugter bei einem verminderten Druck von 10 mbar.

Ein derartig gestaltets Verfahren ermöglicht die besonders effiziente und schonende Anreicherung des Enantiomerenpaars (I) wie hierin beschrieben. Eine besonders schonende Anreicherung ist dabei von großer Bedeutung, da somit ungewollte Zersetzungen reduziert werden können. Eine schonendere Anreicherung bedeutet in der vorliegenden Erfindung somit, dass sowohl die thermischen als auch die druckbedingten Belastung während der Diastereomerenanreicherung gering sind und die daraus erzeugte diastereomerenangereicherte Riechstoffmischung dabei keine negativen thermischen oder druckbedingten Schädigung erleidet, die sich in einer Zersetzung oder einer Veränderung der Produkteigenschaften wie Farbe, Geruch, Stabilität etc. durch die Bildung von Zersetzungs- und Nebenprodukten bemerkbar machen kann. Derartige Zersetzungs- und Nebenprodukte könnten nachteilig mit dem Riechstoff/der Riechstoffmischung wechselwirken und so die gesamte Produktqualität herabsetzen indem beispielsweise unangenehme Nebengerüche hervorrufen werden, die den charakteristischen Geruchseindruck des Riechstoffs bzw. der Riechstoffmischung verfälschen oder nachteilig beeinflussen oder sich gar nachteilig auf die Stabilität der Riechstoffe und Riechstoffmischungen oder der die Reichstoffe und Reichstoffmischungen enthaltenden Zubereitungen und Produkte auswirken.

Weiterhin betrifft die vorliegende Erfindung ein Verfahren zur Herstellung einer diastereomerenangereicherten Riechstoffmischung nach dem ersten Aspekt, wobei der erste grobe Destillationsschritt (a) eine Dünnschichtdestillation beschreibt.

Das erfindungsgemäße Verfahren eignet sich daher insbesondere für die effiziente Herstellung stark diastereomerenangereicherter und reiner Riech- oder Duftstoffe bzw. Riechstoffmischungen in hohen Ausbeuten durch die Kombination aus schonender und selektiver Herstellung unter milden Bedingungen, welche einen optimierten besonders intensiven, ausgewogenen und natürlichen blumigen Geruch aufweisen. Temperaturempfindliche Stoffe, wie beispielsweise viele Riechstoffe und/oder Aromastoffe dürfen im Allgemeinen nur kurzzeitig auf hohe Temperaturen erhitzt werden, um ungewollten thermischen Zersetzungsprozessen entgegenzuwirken. Klassische Destillationsverfahren führen in der Regel zu einer langen thermischen Belastung der zu destillierenden Komponente, welche sowohl die Ausbeute als auch die Qualität der gewonnen Produkte negativ beeinflussen kann. Effiziente Destillationsverfahren mit hoher Selektivität, d.h. hoher Trennleistung und gleichzeitig kurzen Verweilzeiten sind daher vorliegend bei der Anreicherung besonders bevorzugt. Da der Dünnschichtverdampfer in der vorliegenden Erfindung vorzugsweise im Vakuum betrieben wird, ermöglicht das hierin beschriebene Verfahren die Verwendung geringerer Temperaturen und eignet sich deshalb zur besonders schonenden Abtrennung des Roh-Produktes wie hierin beschrieben. Somit zeichnet sich das erfindungsgemäße Verfahren in seiner Gesamtheit als ein sehr schonendes Verfahren aus, welches ungewollte thermische und druckbedingte Zersetzungen reduziert und mit dessen Hilfe bereits das Roh-Produkt in sehr hohen Reinheiten und Ausbeuten effizient und schonend, d.h. mit hervorragender Produktqualität isoliert werden kann.

Außerdem betrifft eine weitere bevorzugte Ausgestaltung des hierin beschriebenen Verfahrens zur Herstellung einer diastereomerenangereicherten Riechstoffmischung des ersten Aspekts eine Dünnschichtdestillation umfassend zwei Stufen:
- Abtrennung des Lösungsmittels bei einem verminderten Druck von etwa 1 mbar bis 400 mbar, vorzugsweise bei einem verminderten Druck von etwa 100 mbar bis 300 mbar, besonders bevorzugt bei einem verminderten Druck von etwa 150 mbar bis 250 mbar und noch bevorzugter bei einem verminderten Druck von etwa 200 mbar; und
- Extraktion des Roh-Produkts umfassend Verbindungen der allgemeinen Formel (A) bei einem verminderten Druck von etwa 0 mbar bis 100 mbar, vorzugsweise bei einem verminderten Druck von etwa 0 mbar bis 10 mbar, besonders bevorzugt bei einem verminderten Druck von etwa 0 mbar bis 5 mbar; noch bevorzugter bei einem verminderten Druck von etwa 1 mbar.

Ferner wird in einer besonders bevorzugten Ausgestaltung des Verfahrens die erste Stufe der Dünnschichtdestillation bei einer Manteltemperatur von zwischen 120 °C und 200 °C durchgeführt, vorzugsweise allerdings bei Temperaturen zwischen 150 °C und 180 °C, noch bevorzugter bei Temperaturen zwischen 160 °C und 175 °C und besonders bevorzugt bei einer Temperatur von etwa 165 °C, und die zweite Stufe der Dünnschichtdestillation bei einer Manteltemperatur von zwischen 150 °C und 250 °C, vorzugsweise allerdings bei Temperaturen zwischen 180 °C und 210 °C, noch bevorzugter bei Temperaturen zwischen 185 °C und 200 °C und besonders bevorzugt bei einer Temperatur von etwa 190 °C.

So lässt sich eine noch schonendere und bezüglich Reinheit und Ausbeute effizientere Extraktion des Magnolan-Roh-Produktes erzielen.

In einem zweiten Gegenstand umfasst die vorliegende Erfindung eine (vorzugsweise racemisch) diastereomerenangereicherte Riechstoffmischung umfassend Verbindungen der allgemeinen Formel (A): 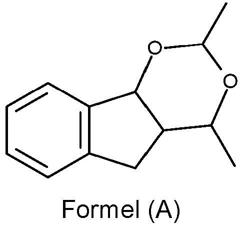 wobei die Verbindungen der Formel (A) die folgenden, zueinander diastereomeren, Enantiomerenpaare der Formeln (I), (II) und (III) umfasst: 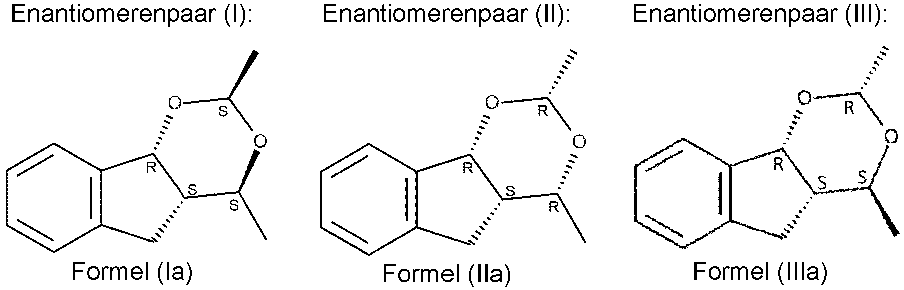 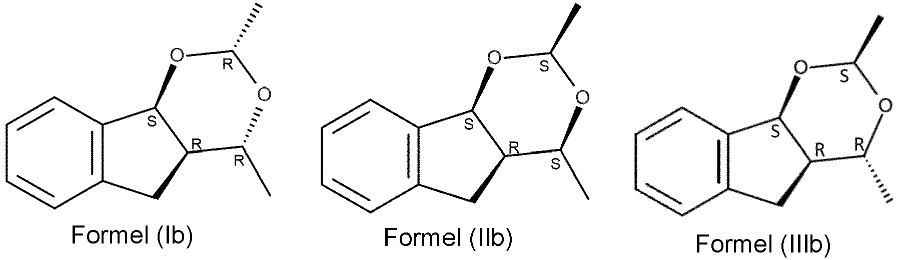 wobei das Mengenverhältnis des Enantiomerenpaars (I) und des Enantiomerenpaars (II) zueinander mindestens 10 : 1 ist und das Mengenverhältnis der Enantiomerenpaare (I) und (III) zueinander mindestens 50 : 1 ist.

Die (vorzugsweise racemisch) diastereomerenangereicherte Riechstoffmischung des zweiten Aspekts, umfassend die Verbindungen der allgemeinen Formel (A), umfasst dabei das Enantiomerenpaar (I) relativ zum Enantiomerenpaar (II) in der Mischung in einem Verhältnis von mindestens 10 : 1. Vorzugsweise, ist das Mengenverhältnis des Enantiomerenpaars (I) und des Enantiomerenpaars (II) in der Mischung zueinander mindestens 15 : 1, bevorzugter mindestens 20 : 1, noch bevorzugter mindestens 50 : 1. Weiter bevorzugt ist das Mengenverhältnis dieser diastereomeren Enantiomerenpaare zueinander mindestens 100 : 1.

Außerdem umfasst die (vorzugsweise racemisch) diastereomerenangereicherte Riechstoffmischung des zweiten Aspekts der vorliegenden Erfindung, umfassend die Verbindungen der allgemeinen Formel (A), die Enantiomerenpaare (I) und (III) zueinander vorzugsweise in einem Mengenverhältnis von mindestens 50:1. Weiter bevorzugt ist das Mengenverhältnis der Enantiomerenpaare (I) und (III) zueinander mindestens 100: 1; noch bevorzugter mindestens 1000 : 1 und am bevorzugtesten mindestens 1360:1.

Die diastereomerenangereicherte Riechstoffmischung, welche nach dem Verfahren des ersten Aspekts herstellbar ist, weist einen intensiven blumigen, natürlichen, rosigen, transparenten und strahlenden, nach Geranie duftendenden Geruch auf, welcher gegenüber kommerziell erhältlichem Magnolan als weniger technisch wahrgenommen wird und keine an Plastik erinnernde Geruchsnoten zeigt, also "sauberer" ist.

Dementsprechend günstig im Sinne der vorliegenden Erfindung sind daher diastereomerenangereicherte Riechstoffmischungen der allgemeinen Formel (A), die einen höheren Anteil des Enantiomerenpaars (I) relativ zu den Enantiomerenpaaren (II) und/oder (III) aufweisen, besonders bevorzugt in einem Verhältnis von Enantiomerenpaar (I) zu Enantiomerenpaar (II) von mindestens 10 : 1 und einem Verhältnis von Enantiomerenpaar (I) zu Enantiomerenpaar (III) von mindestens 50 : 1 in der Riechstoffmischung. Derartige Riechstoffmischungen weisen verstärkt blumige, natürliche, "saubere", rosige, transparente und strahlende, nach Geranie duftendende Geruchsprofile auf. Weiterhin bevorzugt sind die Mengenverhältnisse wie oben definiert.

In einer weiteren bevorzugten Ausgestaltung weist die diastereomerenangereicherte Riechstoffmischung bevorzugt das Enantiomerenpaar (I) in der Reichstoffmischung in an einem Anteil von über 95 Gew.-% (im Vergleich zu allen Enantiomerenpaaren der Riechstoffmischung) auf was dazu führt, dass die positiven Geruchskomponenten der Riechstoffmischung deutlich hervorgehoben werden. Eine derartige Riechstoffmischung weist außerdem ein überaus angenehmes und intensiv blumiges und natürliches Geruchsprofil auf, worin keine technischen oder sonst in irgendeiner Form als negativ wahrgenommenen Geruchsnoten zu erkennen sind.

Eine weitere Ausgestaltung der vorliegenden diastereomerenangereicherten Riechstoffmischung betrifft daher eine Riechstoffmischung umfassend mindestens 95 Gew.-% (im Vergleich zu allen Enantiomerenpaaren) des (vorzugsweise racemischen) Enantiomerenpaars (I). Vorzugsweise ist der Anteil des Enantiomerenpaars (I) allerdings mindestens 97,5 Gew.-% und noch bevorzugter mindestens 98,5 Gew.-% bezogen auf die Summe der Enantiomerenpaare (I), (II) und (III) der erfindungsgemäßen Riechstoffmischung, sodass besonders intensiv-blumige, "saubere" und natürliche Geruchseindrücke erzielt werden können.

Weiterhin zeigt die diastereomerenangereicherte Riechstoffmischung eine hohe Reinheit und kann in sehr hoher Ausbeute bereitgestellt werden.

**In** einer weiteren bevorzugten Ausgestaltung betrifft die vorliegende Erfindung dementsprechend die diastereomerenangereicherte Riechstoffmischung des zweiten Aspekts, wobei die Riechstoffmischung insgesamt eine chemische Reinheit von mindestens 96,5 Gew.-% an den Enantiomerenpaaren (I), (II) und (III) umfasst, bevorzugt allerdings mindestens 98,5 Gew.-%, am bevorzugtesten mindestens 99,0 Gew.-%.

Derartige Riechstoffmischungen weisen ausgezeichnete Sekundäreigenschaften auf, wie beispielsweise eine hohe Stabilität.

**In** einer weiteren optionalen Ausgestaltung stellt der Rest der diastereomerenangereicherten Riechstoffmischung weitere Verunreinigungen dar, wobei die erfindungsgemäße Riechstoffmischung allerdings, weniger als 3,0 Gew.-%, vorzugsweise weniger als 2,0 Gew.-% an derartigen Verunreinigungen enthält, bevorzugt höchstens oder weniger als 1,5 Gew.-%, am bevorzugtesten höchstens 1,0 Gew.-% an derartigen Verunreinigungen bezogen auf die Gesamtmasse der Riechstoffmischung.

Die diastereomerenangereicherten Riechstoffmischungen eignen sich als Riechstoffe bzw. als Zusätze von Riechstoffzubereitungen. Weitere Verwendung finden diese diastereomerenangereicherten Riechstoffmischungen in Konsumgütern enthaltend diese Verbindungen und Mischungen.

In einem dritten Aspekt betrifft die vorliegende Erfindung die Verwendung der diastereomerenangereicherten Riechstoffmischung als Riechstoff oder zur Herstellung einer Riechstoffzubereitung. Somit betrifft die vorliegende Erfindung die Verwendung der erfindungsgemäßen Riechstoffmischung als Riechstoff, insbesondere mit optimiertem, intensive strahlendem, natürlichem und transparentem blumigen Duft, oder zur Herstellung einer Riechstoffzubereitung, insbesondere mit einem derartigen optimierten blumigen Duftprofil.

Ein weiterer Gegenstand der vorliegenden Erfindung betrifft somit eine Riechstoffzubereitung enthaltend eine sensorisch wirksame Menge der diastereomerenangereicherten Riechstoffmischung, wie oben definiert.

Eine Riechstoffzubereitung ist im Rahmen der vorliegenden Erfindung eine Mischung verschiedener Stoffe, welche gemäß einem Rezept oder einer Rezeptur nach einem vorgegebenen Verfahren aus den entsprechenden Stoffen erzeugt wird. Solche Zubereitungen werden gezielt zu dem Zweck hergestellt und eingesetzt, einen gewünschten, üblicherweise als angenehm oder in einer sonstigen Weise positiv empfundenen Geruchseindruck zu vermitteln, zu modifizieren oder zu verstärken.

Eine erfindungsgemäße Riechstoffzubereitung, insbesondere in Form eines Parfümöls, vorzugsweise mit einer optimierten blumigen Geruchsnote wie hierin beschrieben, besteht aus oder umfasst die erfindungsgemäße diastereomerenangereicherte Riechstoffmischung, wie oben definiert, und einen, zwei, drei, vier, fünf, sechs, sieben, acht, neun, zehn oder mehr weitere Riechstoff(e). Auf diese Weise lassen sich einfach Mischungen mit besonders interessanten, intensiven und natürlichen blumigen Geruchsnoten kreieren, welche nicht wie die meisten bisherigen Magnolan-basierten Riechstoffzubereitungen technische oder an Plastik erinnernde Geruchsnoten aufweisen. Die erfindungsgemäßen Riechstoffmischungen können dabei als Einzelstoff oder mit einer Vielzahl weiterer Riechstoffe kombiniert in zahlreichen Produkten verwendet werden, um einen besonderen Geruchseindruck zu kreieren bzw. zu erzeugen.

Riechstoffe und/oder Aromastoffe, die im Sinne der obigen Definition als weitere Riechstoffe oder Aromastoffe für den Einsatz in einer erfindungsgemäßem Riechstoffzubereitung geeignet sind, finden sich z.B. in S. Arctander, "Perfume and Flavor Materials", Vol. I und II, Montclair, N. J. 1969, Eigenverlag, oder K. Bauer et al., "Common Fragrance and Flavor Materials", 4. Edition, Wiley-VCH, Weinheim 2001.

Im Einzelnen seien genannt: Extrakte aus natürlichen Rohstoffen wie Etherische Öle, Concretes, Absolues, Resine, Resinoide, Balsame, Tinkturen wie z.B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamottenöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernnadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole, wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale, wie z.B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-9-undecenal; 2,6,1 0-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime, wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen, wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile, wie z.B. 2-Nonensäurenitril; 2-Undecensäurenitril; 2-Tridecensäurenitril; 3,12-Tridecadiensäurenitril; 3,7 -Dimethyl-2,6-octadien-säurenitril; 3,7-Dimethyl-6-octensäurenitril;
der aliphatischen Carbonsäuren und deren Ester, wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat;
Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat; 4-Methyl-2-pentyl-crotonat;
der acyclischen Terpenalkohole, wie z.B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone, wie z.B. Citronellal; 7-Methoxy-3,7-dimethyloctanal; 2,6,10-Trimethyl-9-undecenal; Geranylaceton; sowie die Dimethyl- und Diethylacetale von Geranial, Neral;
der cyclischen Terpenalkohole, wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone, wie z.B. Menthon; Isomenthon; 8-Mercaptomenthan-3-on; Carvon; Campher; Fenchon; alpha-Ionon; beta-lonon; alphan-Methylionon; beta-n-Methylionon; alpha-Isomethylionon; beta-Isomethylionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8-(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole, wie z.B. 4-tert-Butylcyclohexanol; 3,3,5-Trimethylcyclohexanol; 3-Isocamphylcyclohexanol; 2,6,9- T rimethyl-Z2,Z5, E9-cyclododecatrien-1-ol; 2-Isobutyl-4-methyltetrahydro-2H-pyran-4-ol;
der cycloaliphatischen Alkohole, wie z.B. alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether, wie z.B. Cineol; Cedrylmethylether; Cyclododecylmethylether; 1,1-Dimethoxycyclododecan; (Ethoxymethoxy)-cyclododecan; alpha-Cedrenepoxid; 3a,6,6,9a-Tetramethyldodecahydro-naphtho[2,1b]furan; 3a-Ethyl-6,6,9a-trimethyldodecahydronaphtho[2,1b]furan; 1,5,9-Trimethyl-13-oxabicyclo[10.1.0]trideca-4,8-dien; Rosenoxid; 2-(2,4-Dimethyl-3-cyclohexen-1-yl)-5methyl-5-(1-methylpropyl)-1,3-dioxan;
der cyclischen und makrocyclischen Ketone, wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde, wie z.B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone, wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon;tert.-Butyl-(2,4-dimethyl-3-cyclohexen-1-yl)keton;
der Ester cyclischer Alkohole, wie z.B. 2-tert-Butylcyclohexylacetat; 4-tert.-Butylcyclohexylacetat; 2-tert-Pentylcyclohexylacetat; 4-tert.-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5- bzw. 6-inden-ylisobutyrat; 4,7-Methanooctahydro-5- bzw. 6-indenylacetat;
der Ester cycloaliphatischer Alkohole, wie z.B. 1-Cyclohexylethylcrotonat;
der Ester cycloaliphatischer Carbonsäuren, wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der aliphatischen Alkohole, wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von aliphatischen Alkoholen und aliphatischen Carbonsäuren, wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der aliphatischen Ether, wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan;
der aromatischen und aliphatischen Aldehyde, wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butylphenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal;-Methyl-3-(4-methylendioxyphenyl)propanal;
der aromatischen und aliphatischen Ketone, wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon; (3-Methyl-2-benzofuranyl)ethanon; Benzophenon; 1,1,2,3,3,6-Hexamethyl-5-indanylmethylketon; 6-tert.-Butyl-1,1-di-methyl-4-indanylmethylketon; 1-[2*,3*-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und aliphatischen Carbonsäuren und deren Ester, wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnamat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen, wie z.B. 2,4,6-Trinitro-1,3-dimethyl-5-tert.-butylbenzol; 3,5-Dinitro-2,6-dimethyl-4-tert.-butylacetophenon; Zimtsäurenitril;
3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropyl-chinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester, wie z.B. Estragol; Anethol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen, wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone, wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; 2,3-Dihydrocumarin; Octahydrocumarin.

In einer erfindungsgemäßen Riechstoffzubereitung, insbesondere eines Parfümöls, liegt die Menge der diastereomerenangereicherten Riechstoffmischung, wie oben definiert, in einem Bereich von 0,0001 Gew.-% bis 40 Gew.-%, vorzugsweise im Bereich von 0,001 Gew.-% bis 25 Gew.-%, und besonders bevorzugt in einem Bereich von 0,0001 Gew.-% bis 10 Gew.-% bzw. 5 Gew.-% bezogen auf das Gesamtgewicht der Riechstoffzubereitung.

Neben dem Einsatz als Flüssigkeit in Lösungen oder in Emulsionen können die erfindungsgemäßen Riechstoffmischungen oder Riechstoffzubereitungen enthaltend diese erfindungsgemäßen Riechstoffmischungen, beispielsweise Parfümöle, an Feststoffen adsorbiert oder in Trägerstoffen (mikro)verkapselt eingesetzt werden. Diese Darreichungsformen können sowohl für eine feine Verteilung der Riechstoffe im Produkt als auch für eine kontrollierte Freisetzung bei der Anwendung sorgen. Derartige Feststoffe können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker oder Kunststoffe wie PVC, Polyvinylacetate oder Polyurethane sein.

Verkapselungsprodukte können beispielsweise sprühgetrocknet, als Einschluss-Komplex oder als Extrusions-Produkt vorliegen.

Unter einer "sensorisch wirksamen Menge" ist im Kontext der vorliegenden Erfindung zu verstehen, dass der Riechstoff bzw. die diastereomerenangereicherte Riechstoffmischung in solch einer ausreichenden Menge vorhanden ist, dass das daraus resultierende Produkt bei Verwendung die sensorischen Eigenschaften des Riechstoffs bzw. der erfindungsgemäßen Riechstoffmischung erkennen lässt. So ist unter einer sensorisch wirksamen Menge ein Anteil an der erfindungsgemäßen Riechstoffmischung zu verstehen, der ausreichend ist, um die hierin Effekte, d.h. beispielsweise Hervorheben bzw. Betonen einer angenehmen und optimierten blumigen Geruchsnote und/oder einen maskierenden Effekt zu bewirken.

Ferner betrifft die vorliegende Erfindung somit die Verwendung der diastereomerenangereicherten Riechstoffmischung nach einem der vorangehenden Aspekte in einer sensorisch wirksamen Menge zum Vermitteln von, Modifizieren oder Verstärken einer blumigen Geruchsnote eines parfümierten Produktes oder zur Herstellung eines parfümierten Produktes. Verstärken bedeutet in diesem Kontext eine bestimmte Duftnote hervorzuheben oder zu betonen, insbesondere von Gerüchen bzw. Geruchsnoten des wie hierin beschriebenen optimierten intensiven und natürlich "sauberen" blumigen Typs.

Da in der Parfümerie-Branche insbesondere Blumenriechstoffe, d.h. Riechstoffe mit einer"sauberen" blumigen Note besonders bevorzugt und gefragt sind, eignen sich die in der vorliegenden Erfindung beschriebenen Riechstoffe und Riechstoffzubereitungen umfassend die diastereomerenangereicherte Riechstoffmischung besonders für derartige Verwendungen. Dabei werden insbesondere Duftnoten des technischen Typs bzw. die an Plastik erinnern in der Regel als negativ und daher als störend empfunden. Da die vorliegende Riechstoffmischung einfach, in wenigen Schritten und gleichzeitig in hoher Ausbeute und in hoher Reinheit bereitgestellt werden kann, kann eine Riechstoffmischung zur Verfügung gestellt werden, welche die besagten als negativ empfundenen Geruchsnoten nicht aufweist und sich somit für eine Vielzahl von Anwendungen eignet, insbesondere für das Vermitteln, Modifizieren oder Verstärken einer intensiv strahlenden und "sauberen" blumigen Geruchsnote.

Unter "sauber" ist vorliegend eine Geruchsnote zu verstehen, welche keine technischen, an Plastik erinnernden oder in einer sonstigen Weise negativ empfundenen und von der blumigen Note ablenkenden und nicht als natürlich empfundene Geruchscharakteristika aufweist.

Die hierin beschriebenen Riechstoffe bzw. Riechstoffmischungen und Zubereitungen zeigen eine deutliche Verstärkung der intensiven, natürlichen und "sauberen" blumigen Duftnoten bereits bei geringen Dosierungen.

Es konnte überraschend beobachtet werden, dass die hierin beschriebene Riechstoffmischung ausgezeichnete Stabilitätseigenschaften aufweist und sich somit hervorragend in eine Vielzahl von Produktformulierungen einarbeiten lässt. Folglich eignet sich die vorliegende Riechstoffmischung insbesondere für die Herstellung einer Vielzahl von parfümierten Produkten, wie beispielsweise Shampoos, Cremes, Seifen, Deodorantien, Reinigungsmitteln und dergleichen.

Letztlich betrifft die vorliegende Erfindung daher ein parfümiertes Produkt, umfassend die diastereomerenangereicherten Riechstoffmischung, wie oben beschrieben, oder eine entsprechende Riechstoffzubereitung in konzentrierter Form, in Lösungen oder in sonstiger modifizierter Form für die Herstellung von Konsumgütern oder parfümierten Produkten im Sinn der Erfindung, wie z.B. Parfüm-Extraits, Eau de Parfums, Eau de Toilettes, Rasierwässern, Eau de Colognes, Preshave-Produkten, Splash-Colognes und parfümierten Erfrischungstüchern sowie für die Parfümierung von sauren, alkalischen und neutralen Reinigungsmitteln, wie z.B. Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmitteln, Einweichmitteln und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-Cremes und -Lotionen, Handcremes und -lotionen, Fußcremes und -lotionen, Enthaarungscremes und -lotionen, Aftershave-Cremes und -Lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

Folglich betrifft die vorliegende Erfindung letztlich auch ein parfümiertes Produkt, umfassend die diastereomerenangereicherten Riechstoffmischung, wie oben beschrieben, oder eine entsprechende Riechstoffzubereitung diese umfassend.

### Beispiele

Die vorliegende Erfindung wird nachstehend anhand von Ausführungsbeispielen näher beschrieben.

Herstellung einer diastereomerenangereicherten Riechstoffmischung entsprechend dem hierin beschriebenen Verfahren:
Zunächst wurde eine Emulsion aus verdünnter Schwefelsäure und Toluol in einem Reaktionsgefäß vorgelegt. Anschließend wurden bei einer Temperatur von 5 °C die Reaktanden Paraldehyd und Inden hinzugegeben und für eine Dauer von 5 Stunden gerührt. Anschließend wurde das Reaktionsprodukt dreimal mit Wasser gewaschen und die organische Phase abdestilliert.

Entsprechend Schritt (a) des hierin beschriebenen Verfahrens wurde das daraus gewonnen Reaktionsprodukt einem Dünnschichtverdampfer zugeführt. In der ersten Stufe der Dünnschichtdestillation wurde das Roh-Produkt von etwaigen Lösungsmittelrückständen und nicht umgesetzten Edukten bei 200 mbar und einer Manteltemperatur von 165 °C befreit. Durch Destillation bei 1 mbar und einer Manteltemperatur von 190 °C erfolgte anschließend die Abnahme des aufgereinigten Roh-Produktes (zweite Stufe der Dünnschichtdestillation).

Anschließend wurde das so erhaltene Roh-Produkt einer Feindestillation unterworfen (Schritt (b)). Hierzu erfolgte eine Destillation des Roh-Produkts in einer Destillationsapparatur mit Sulzer BX als Füllmaterial über 20 Trennstufen in einem kontinuierlichen Prozess. Das Rücklaufverhältnis betrug dabei in etwa 10 : 1, die Kopftemperatur etwa 138 °C bis 139 °C und die Destillation wurde bei einem verminderten Druck von etwa 10 mbar durchgeführt.

Das so erhaltene Produkt weist die Enantiomerenpaare in den folgenden Mengenverhältnissen auf: das Mengenverhältnis von Enantiomerenpaar (I) zu Enantiomerenpaar (II) in der erhaltenen Riechstoffmischung beträgt 96,894 zu 0,577 während das Mengenverhältnis des Enantiomerenpaars (I) zu dem Enantiomerenpaar (III) 96,894 zu 0,047 beträgt. Folglich ist auch der Anteil des Enantiomerenpaars (I) im Vergleich zu der Gesamtheit der Verbindungen nach Formal (A), also bezogen auf die Summe der Enantiomerenpaare (I), (II) und (III), in der so erhaltenen Riechstoffmischung mindestens 95 Gew.-% und weist eine entsprechende Diastereomerenreinheit auf. In diesem Fall betrug die chemische Reinheit, d.h. die Summe aller Diastereomere der Formel (A) im Verhältnis zu allen anderen Verunreinigungen 97,5 zu 2,5.

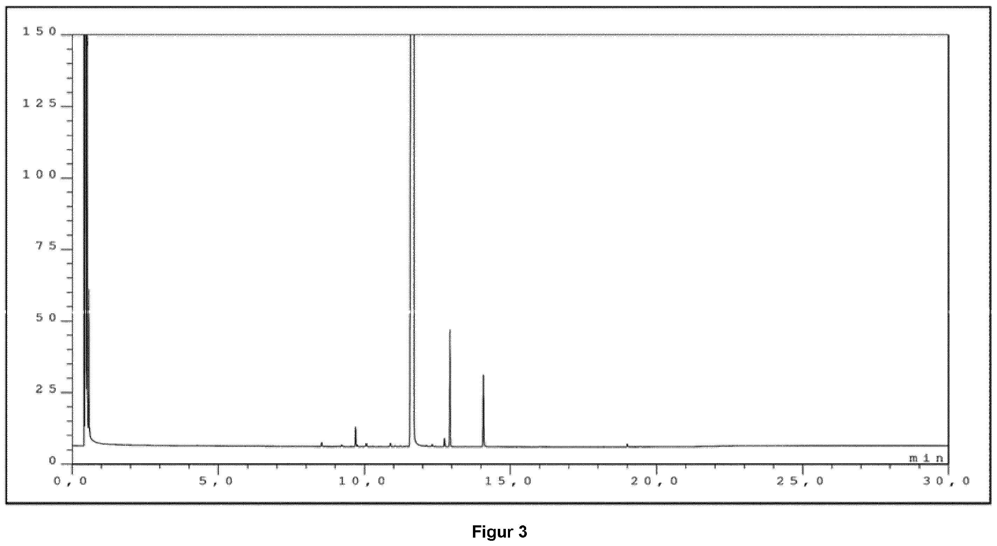

In einem weiteren zweiten Beispiel nach gleichem Verfahren betrug das Verhältnis der Enantiomerenpaare (I) zu (II) zu (III) 97,971 zu 0,979 zu 0,072. Auch hier liegt der Anteil des Enantiomerenpaars (I) bei über 95 Gew.-% bezogen auf die Summe der Enantiomerenpaare (I), (II) und (III). Zudem weist das erhaltene Produkt eine chemische Reinheit von über 98,5 Gew.-% auf, d.h. es sind weniger als 1 Gew.- % an Verunreinigungen enthalten (siehe Figur 3).

### Analytik:

Eine gaschromatographische Analyse des ersten Beispiels ergab eine Zusammensetzung von 96,894 Gew.-% des Enantiomerenpaars (I), 0,577 Gew.-% des Enantiomerenpaars (II) und 0,047 Gew.-% des Enantiomerenpaars (III). In dem zweiten Beispiel konnte eine Zusammensetzung entsprechend den folgenden Anteilen nachgewiesen werden: 97,971 % des Enantiomerenpaars (I), 0,979 % des Enantiomerenpaars (II) und 0,072 % des Enantiomerenpaars (III).

Die entsprechenden Werte wurden mittels Gaschromatographen der Marke Thermo Fisher Scientific (TRACE1300Series), wobei als Säulenmaterial Polyethylenglycol verwendet wurde.

Entsprechende NMR-Messungen des ersten Beispiels und eine gaschromatographische Analyse des zweiten Beispiels sind den Figuren zu entnehmen.

NMR-Daten des ersten Beispiels:
H-NMR: 7.38 m (1H), 7.25 m (2H), 7.21 m (1H), 5.46 d, J=6.60 (1H), 4.75 q, J=5.09 (1H), 3.32 dq, J= 10.38, 6.13 (1H), 2.92 ddd, J=15.89, 6.52, 1.25 (1H), 2.49 d,
J=16.00 (1H), 2.40 dt, J=10.44, 6.58 (1H), 1.35 d, J=5.11 (3H), 1.27 d, J=6.15 (3H).

Ferner weist die erhaltene Riechstoffmischung keinen messbaren Drehwert auf, sodass darauf geschlossen werden kann, dass die Enantiomere der jeweiligen Enantiomerenpaare jeweils in racemischer Form zueinander vorliegen, d.h. die Enantiomerenpaare (I), (II) und (III) sind jeweils racemisch.

### Vergleichsbeispiel:

Während die Isomere (I) und (II) in einem Verhältnis von mindestens 10 : 1 zueinander vorliegen, zeigt ein Vergleichsbeispiel, dass bereits die Veränderungen des Rücklaufverhältnisses auf 1 : 1 und der Kopftemperatur auf etwa 141 °C bis 142 °C bei sonst gleichen Destillationsbedingungen zu einem Mengenverhältnis des Enantiomerenpaars (I) zu dem Enantiomerenpaar (II) in der Riechstoffmischung von lediglich 1 : 2 bis 2 : 1 führt.

Folglich war es möglich, durch geschickte Wahl der Destillationsbedingungen eine erfindungsgemäße diastereomerenangereicherte Riechstoffmischung bereitzustellen, welche den präferierten und optimierten blumigen Duft aufweist.

### Geruchsbeschreibung:

Für die Bestimmung der olfaktorischen Eigenschaften, wurden die einzelnen Enantiomerenpaare (I), (II) und (III) durch chromatographische Trennung separat erhalten und hinsichtlich ihres Geruchs von einem Testpanel von 8 Testpersonen bewertet: Die einzelnen Enantiomerenpaare weisen dabei die folgenden Geruchsprofile auf: Der Geruch des Enantiomerenpaars (I) kann im Wesentlichen als blumig, rosig, transparent, strahlend und nach Geranium riechend beschrieben werden, während das Enantiomerenpaar (II) einen blumigen, grünen, etwas technischen, nach Grapefruit riechenden und im Vergleich zum Enantiomerenpaar (I) schwächeren Geruchseindruck erweckt. Das Enantiomerenpaar (III) hingegen weist im Wesentlichen ein blumiges, technisches, unsauberes und nach Plastik riechendes Geruchsprofil auf.

Die Ergebnisse des Tests der olfaktorischen Eigenschaften sind in der folgenden Tabelle 1 dargestellt:

**Tabelle 1:**

| **Duftbeschreibung** | Kommerziell erhältliches Magnolan | Isomerenmischung nach Anspruch 1 | Enantiome ren-paar 1 | Enantiomer en-paar 2 | Enantiome ren-paar 3 |
|---|---|---|---|---|---|
| Rose | 6 | 8 | 8 | 5 | 4 |
| Maiglöckchen | 6 | 5 | 6 | 7 | 3 |
| Orris / Veilchen | 2 | 0 | 0 | 2 | 2 |
| Blattgemüse | 5 | 2 | 2 | 6 | 5 |
| Jasmin / Ylang | 3 | 2 | 2 | 3 | 2 |
| Orangenblüte | 3 | 1 | 1 | 3 | 2 |
| Fruchtig | 5 | 3 | 2 | 6 | 5 |
| Indolartig | 3 | 0 | 0 | 2 | 5 |
| Plastik | 3 | 1 | 0 | 1 | 6 |

Der Tabelle 1 ist zu entnehmen, dass das Enantiomerenpaar (III) und das kommerziell erhältliche Magnolan, welches in dem Vergleichsbeispiel hergestellt wurde, einen starken Plastikgeruch aufweisen. Im Gegensatz dazu, weist die Isomerenmischung der vorliegenden Erfindung nach Anspruch 1 einen deutlich niedrigeren Plastikgeruch, sowie eine strahlendere Rosennote auf. Somit konnte der Geruch des kommerziell erhältlichen Magnolan verbessert werden.

Der Geruch des insgesamt racemischen Produkts, d.h. der erfindungsgemäßen Riechstoffmischung, nach dem erfindungsgemäßen Beispielen konnte in beiden Fällen als etwas wärmer, intensiver und natürlicher blumig, rosig, transparent, strahlend und nach Geranium riechend beschrieben werden, während Vergleichsriechmischungen mit von der vorliegenden Beschreibung abweichenden Enantiomerenzusammensetzungen geprägt sind von den geruchlichen Eigenschaften der Enantiomerenpaare (II) und/oder (III) und folglich einen etwas technischen, nach Grapefruit riechenden und im Vergleich zum Enantiomerenpaar (I) schwächeren, d.h. weniger intensiven, unsauberen und insgesamt weniger blumigen Geruchseindruck erweckten, der teilweise sogar intensive technische oder gar an Plastik erinnernde und unsaubere Noten aufweist. Daher sind die aufgereinigten Produkte nach der Erfindung vorzuziehen.

Kommerziell erhältliche Magnolan-Produkte, wie auch das im Vergleichsbeispiel hergestellte Magnolan oder das Magnolan-Roh-Produkt weisen ebenfalls einen etwas technischen und nach Plastik riechenden Geruch auf, welches somit als blumig, grün, etwas rosig, etwas technisch und nach Grapefruit und Plastik riechend beschrieben werden kann.

**Im** Gegensatz dazu zeigen die aus dem hierin beschriebenen Verfahren erhaltenen diastereomerenangereicherten Riechstoffmischungen des ersten und zweiten Aspekts, wie hierin beschrieben, einen überaus intensiv strahlenden, ausgewogenen, natürlichen, "sauberen" blumigen, rosigen, transparenten, etwas wärmeren und nach Geranie duftenden Geruch auf, welcher im Allgemeinen als angenehmer, balancierter und "sauberer" gegenüber den obig genannten Produkten empfunden wird, und keine technischen oder an Plastik erinnernden Geruchsnoten aufweist.

Entsprechend konnte mit dem hierin beschrieben Verfahren ein effizientes, einfaches und schonendes destillatives Verfahren zur effektiven und schonenden destillativen Anreicherung des Enantiomerenpaars (I) und die Bereitstellung der diastereomerenangereicherten Riechstoffmischung umfassend die Verbindungen der allgemeinen Formel (A) gemäß dem ersten und zweiten Aspekt in hoher Reinheit und Ausbeute gezeigt werden. Insbesondere war es überraschend gelungen, das Enantiomerenpaar (I) in einem Anteil von über 95 Gew.-% bezogen auf die Summe der Enantiomerenpaare (I), (II) und (III) in der erfindungsgemäßen Riechstoffmischung anzureichern und so den erzeugten Geruchseindruck effektiv zu optimieren.

## Patentansprüche

1. Destillatives Verfahren zur Herstellung einer diastereomerenangereicherten Riechstoffmischung umfassend Verbindungen der allgemeinen Formel (A): 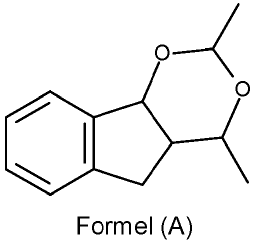
wobei die Riechstoffmischung die folgenden, zueinander diastereomeren, Enantiomerenpaare umfasst: 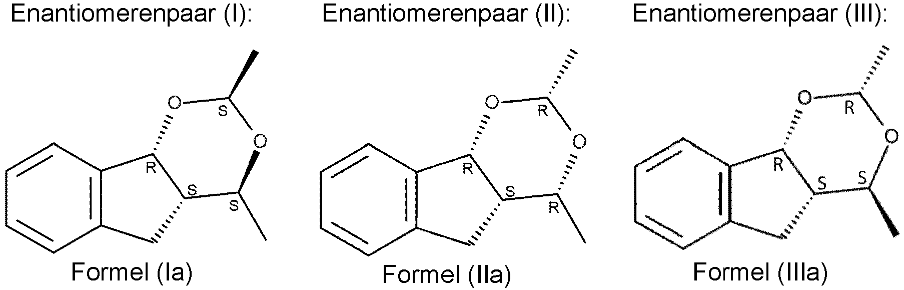 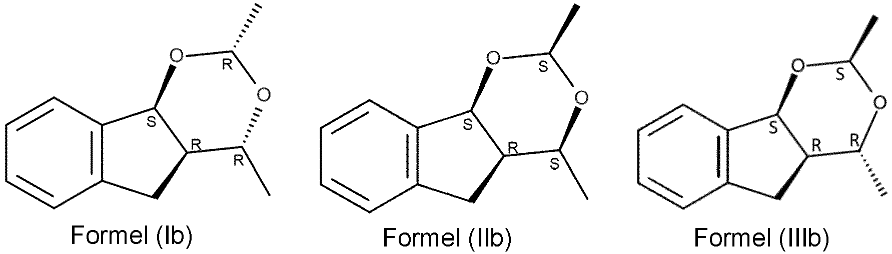
bei der das Mengenverhältnis des Enantiomerenpaars (I) und des Enantiomerenpaars (II) zueinander mindestens 10 : 1;
wobei das Mengenverhältnis der Enantiomerenpaare (I) und (III) zueinander mindestens 50 : 1 ist; und
wobei das Verfahren die folgenden destillativen Schritte umfasst:
(a) destillative Abtrennung eines Roh-Produkts umfassend Verbindungen der allgemeinen Formel (A) in einem ersten Destillationsschritt;
(b) anschließende Feindestillation des Roh-Produkts über einen oder mehrere Destillationsschritte unter Aufkonzentrierung des Enantiomerenpaars (I) im Verhältnis zu den Enantiomerenpaaren (II) und (III), wobei die Feindestillation mindestens 15 Trennstufen umfasst.

2. Verfahren zur Herstellung einer diastereomerenangereicherten Riechstoffmischung nach Anspruch 1, vor den destillativen Schritten weiterhin umfassend:
- die Bereitstellung von Paraldehyd der allgemeinen Formel (IV) oder Acetaldehyd; 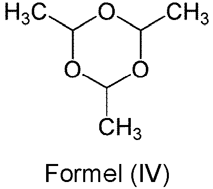 sowie
- dessen Umsetzung mit Inden der allgemeinen Formel (V) unter saurer Katalyse im Lösungsmittel, 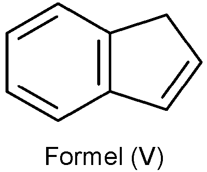 wobei die Umsetzung des Paraldehyds mit Inden bei Temperaturen unterhalb 10 °C erfolgt; und
- Gewinnung des Roh-Produkts umfassend Verbindungen der allgemeinen Formel (A).

3. Verfahren zur Herstellung einer diastereomerenangereicherten Riechstoffmischung nach einem der vorangehenden Ansprüche 1 oder 2, wobei die Feindestillation des destillativen Verfahrens in einem kontinuierlichen Prozess erfolgt.

4. Verfahren zur Herstellung einer diastereomerenangereicherten Riechstoffmischung nach einem der vorangehenden Ansprüche 1 bis 3, wobei die Feindestillation des destillativen Verfahrens ein Rücklaufverhältnis von mindestens 5 : 1 aufweist.

5. Verfahren zur Herstellung einer diastereomerenangereicherten Riechstoffmischung nach einem der vorangehenden Ansprüche 1 bis 4, wobei die Feindestillation des destillativen Verfahrens bei Temperaturen zwischen 120 °C und 150 °C durchgeführt wird.

6. Verfahren zur Herstellung einer diastereomerenangereicherten Riechstoffmischung nach einem der vorangehenden Ansprüche 1 bis 5, wobei die Feindestillation des destillativen Verfahrens bei einem verminderten Druck von etwa 1 bis 100 mbar durchgeführt wird.

7. Verfahren zur Herstellung einer diastereomerenangereicherten Riechstoffmischung nach einem der vorangehenden Ansprüche 1 bis 6, wobei der erste Destillationsschritt eine Dünnschichtdestillation ist.

8. Verfahren zur Herstellung einer diastereomerenangereicherten Riechstoffmischung nach Anspruch 7, wobei die Dünnschichtdestillation zwei Stufen umfasst:
- Abtrennung des Lösungsmittels bei einem verminderten Druck von etwa 1 mbar bis 400 mbar; und
- Extraktion des Roh-Produkts umfassend Verbindungen der allgemeinen Formel (A) bei einem verminderten Druck von etwa 0 mbar bis 100 mbar.

9. Verfahren zur Herstellung einer diastereomerenangereicherten Riechstoffmischung nach einem der vorangehenden Ansprüche 7 oder 8, wobei die erste Stufe der Dünnschichtdestillation bei einer Manteltemperatur von zwischen 120 °C und 200 °C durchgeführt wird und wobei die zweite Stufe der Dünnschichtdestillation bei einer Manteltemperatur von zwischen 150 °C und 250 °C durchgeführt wird.

10. Diastereomerenangereicherte Riechstoffmischung umfassend Verbindungen der allgemeinen Formel (A): 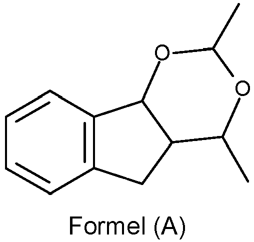
wobei die Verbindungen der Formel (A) die folgenden, zueinander diastereomeren, Enantiomerenpaare der Formeln (I), (II) und (III) umfasst: 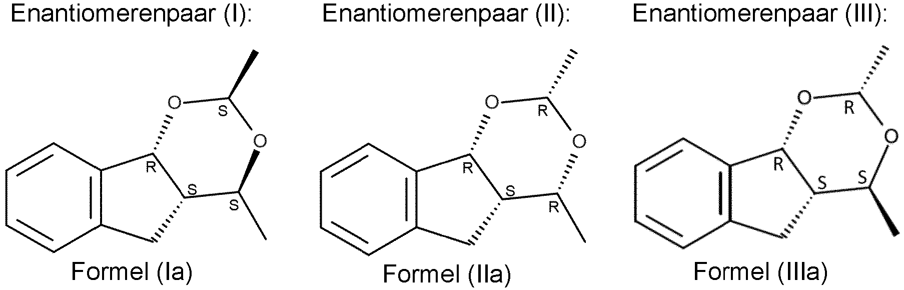 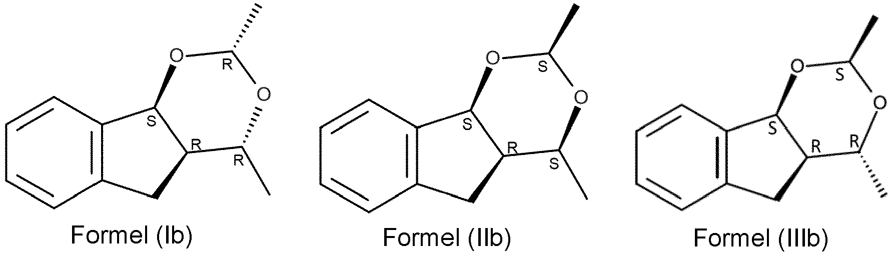
wobei das Mengenverhältnis des Enantiomerenpaars (I) und des Enantiomerenpaars (II) zueinander mindestens 10 : 1 ist; und
wobei das Mengenverhältnis der Enantiomerenpaare (I) und (III) zueinander mindestens 50 : 1 ist.

11. Diastereomerenangereicherte Riechstoffmischung nach Anspruch 10, wobei die Riechstoffmischung insgesamt mindestens 96,5 Gew.-% an den Enantiomerenpaaren (I), (II) und (III) umfasst.

12. Verwendung der diastereomerenangereicherten Riechstoffmischung nach einem der vorangehenden Ansprüche 10 oder 11 als Riechstoff oder zur Herstellung einer Riechstoffzubereitung.

13. Riechstoffzubereitung enthaltend eine sensorisch wirksame Menge der diastereomerenangereicherten Riechstoffmischung nach einem der vorangehenden Ansprüche 10 oder 11.

14. Verwendung der diastereomerenangereicherten Riechstoffmischung nach einem der vorangehenden Ansprüche 10 oder 11 in einer sensorisch wirksamen Menge zum Vermitteln von, Modifizieren oder Verstärken einer blumigen Geruchsnote eines parfümierten Produktes oder zur Herstellung eines parfümierten Produktes.

15. Parfümiertes Produkt, umfassend die diastereomerenangereicherten Riechstoffmischung nach Anspruch 10 oder 11 oder eine Riechstoffzubereitung nach Anspruch 13.

## Claims

1. Distillative method for preparing a diastereomer-enriched fragrance mixture comprising compounds of general formula (A): 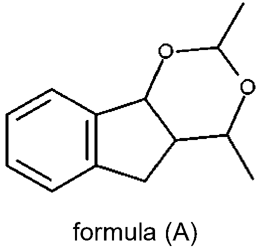
wherein the fragrance mixture comprises the following, mutually diastereomeric, enantiomer pairs: 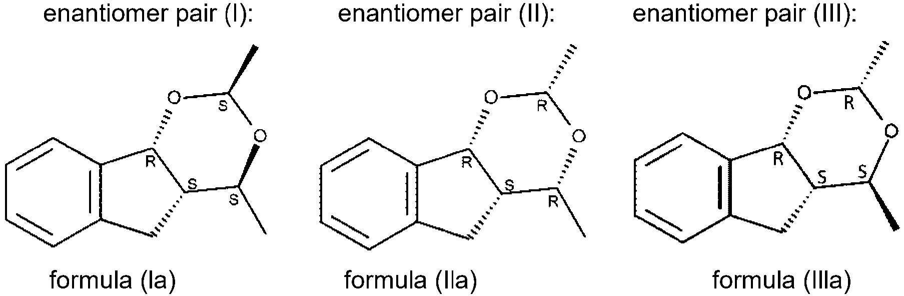 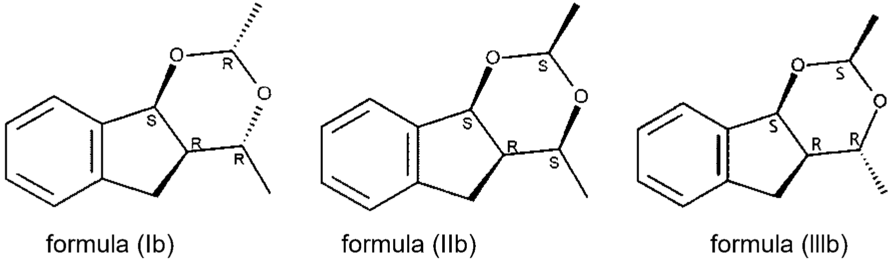
wherein the quantitative ratio between enantiomer pair (I) and enantiomer pair (II) is at least 10:1;
wherein the quantitative ratio between enantiomer pairs (I) and (III) is at least 50:1; and
wherein the method comprises the following distillation steps:
(a) separating off, by distillation, a crude product comprising compounds of general formula (A) in a first distillation step;
(b) then finely distilling the crude product over one or more distillation steps to concentrate enantiomer pair (I) relative to enantiomer pairs (II) and (III), the fine distillation comprising at least 15 separation stages.

2. Method for preparing a diastereomer-enriched fragrance mixture according to claim 1, which further comprises, prior to the distillation steps:
- providing paraldehyde of general formula (IV) or acetaldehyde; 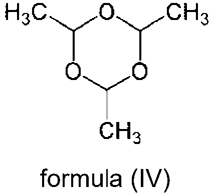 and
- reacting it with indene of general formula (V) under acidic catalysis in a solvent, 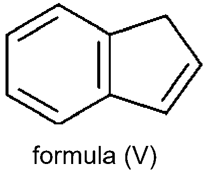 the reaction between paraldehyde and indene taking place at temperatures below 10°C; and
- obtaining the crude product comprising compounds of general formula (A).

3. Method for preparing a diastereomer-enriched fragrance mixture according to any one of the preceding claims 1 or 2, wherein the fine distillation in the distillation method takes place in a continuous process.

4. Method for preparing a diastereomer-enriched fragrance mixture according to any one of the preceding claims 1 to 3, wherein the fine distillation in the distillation method has a reflux ratio of at least 5:1.

5. Method for preparing a diastereomer-enriched fragrance mixture according to any one of the preceding claims 1 to 4, wherein the fine distillation in the distillation method is carried out at temperatures between 120°C and 150°C.

6. Method for preparing a diastereomer-enriched fragrance mixture according to any one of the preceding claims 1 to 5, wherein the fine distillation in the distillation method is carried out at a reduced pressure of about 1 to 100 mbar.

7. Method for preparing a diastereomer-enriched fragrance mixture according to any one of the preceding claims 1 to 6, wherein the first distillation step is a thin film distillation.

8. Method for preparing a diastereomer-enriched fragrance mixture according to claim 7, wherein the thin film distillation comprises two stages:
- separating off the solvent at a reduced pressure of about 1 mbar to 400 mbar; and
- extracting the crude product comprising compounds of general formula (A) at a reduced pressure of about 0 mbar to 100 mbar.

9. Method for preparing a diastereomer-enriched fragrance mixture according to any one of the preceding claims 7 or 8, wherein the first stage of the thin film distillation is carried out at a jacket temperature of between 120°C and 200°C, and wherein the second stage of the thin film distillation is carried out at a jacket temperature of between 150°C and 250°C.

10. Diastereomer-enriched fragrance mixture comprising compounds of general formula (A): 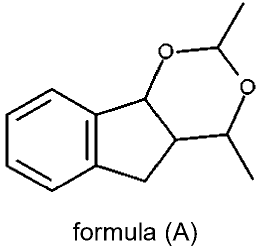
wherein the compounds of formula (A) comprise the following, mutually diastereomeric, enantiomer pairs of formulae (I), (II) and (III): 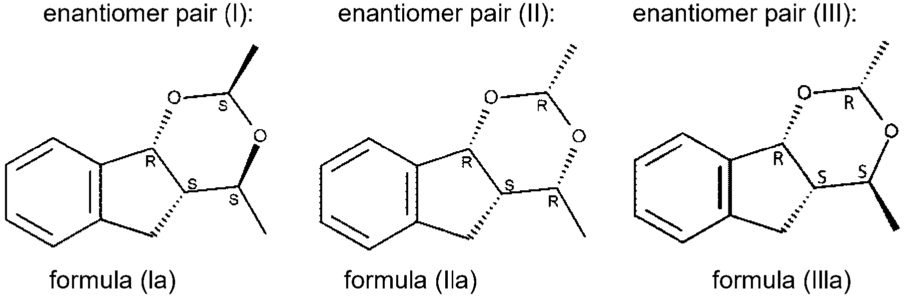 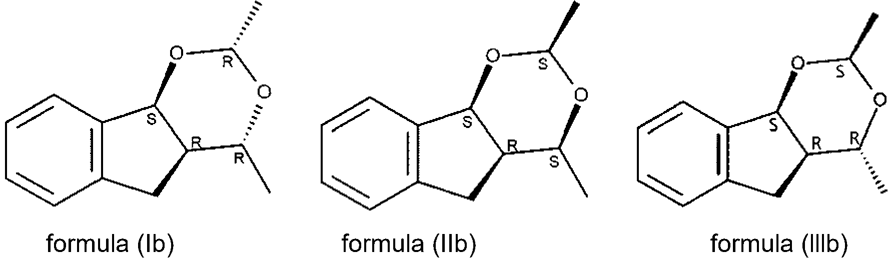
wherein the quantitative ratio between enantiomer pair (I) and enantiomer pair (II) is at least 10:1; and
wherein the quantitative ratio between enantiomer pairs (I) and (III) is at least 50:1.

11. Diastereomer-enriched fragrance mixture according to claim 10, wherein the fragrance mixture comprises, in total, at least 96.5% by weight of enantiomer pairs (I), (II) and (III).

12. Use of the diastereomer-enriched fragrance mixture according to any one of the preceding claims 10 or 11 as a fragrance or for preparing a fragrance composition.

13. Fragrance composition containing a sensorially effective amount of the diastereomer-enriched fragrance mixture according to any one of the preceding claims 10 or 11.

14. Use of the diastereomer-enriched fragrance mixture according to any one of the preceding claims 10 or 11 in a sensorially effective amount for imparting, modifying or enhancing a floral scent of a perfumed product or for preparing a perfumed product.

15. Perfumed product, comprising the diastereomer-enriched fragrance mixture according to claim 10 or 11 or a fragrance composition according to claim 13.

## Revendications

1. Procédé de distillation pour la production d'un mélange de substances odorantes enrichi en diastéréoisomères comprenant des composés de formule générale (A) : 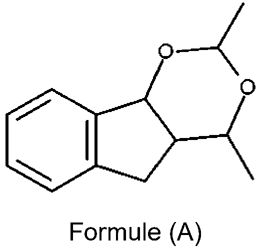
le mélange de substances odorantes comprenant les paires d'énantiomères diastéréoisomères suivantes : 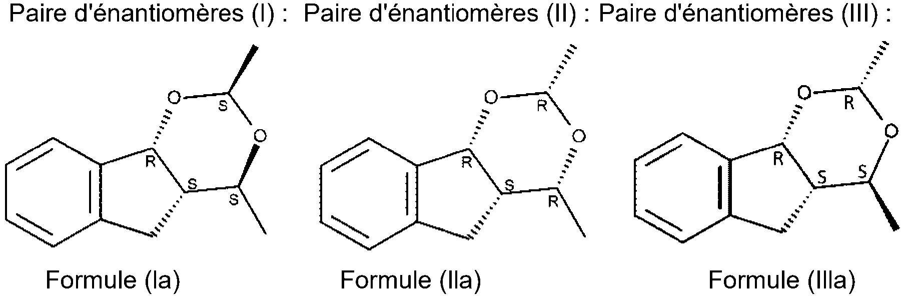 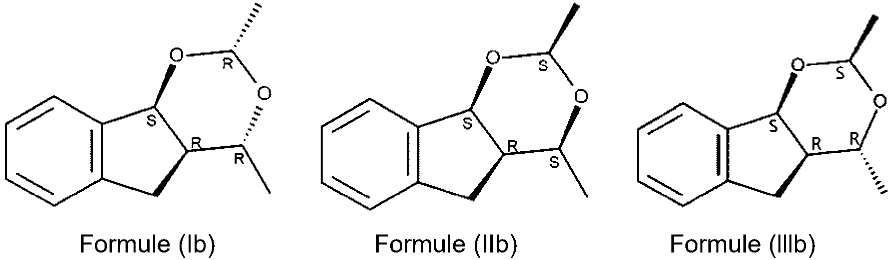
dans lequel le rapport quantitatif entre la paire d'énantiomères (I) et la paire d'énantiomères (II) est d'au moins 10 : 1 ;
le rapport quantitatif entre les paires d'énantiomères (I) et (III) étant d'au moins 50 : 1 ; et
le procédé comprenant les étapes de distillation suivantes :
(a) la séparation par distillation d'un produit brut comprenant des composés de formule générale (A) dans une première étape de distillation ;
(b) la distillation fine subséquente du produit brut en une ou plusieurs étapes de distillation avec concentration de la paire d'énantiomères (I) par rapport aux paires d'énantiomères (II) et (III), la distillation fine comprenant au moins 15 étapes de séparation.

2. Procédé de préparation d'un mélange de substances odorantes enrichi en diastéréoisomères selon la revendication 1, comprenant en outre, avant les étapes de distillation :
- la fourniture de paraldéhyde de formule générale (IV) ou d'acétaldéhyde ; 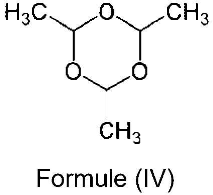 Ainsi que
- sa réaction avec l'indène de formule générale (V) sous catalyse acide dans le solvant, 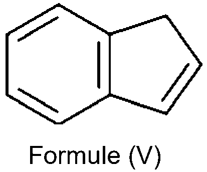 la réaction du paraldéhyde avec l'indène s'effectuant à des températures inférieures à 10 °C ; et
- l'obtention du produit brut comprenant des composés de formule générale (A).

3. Procédé de fabrication d'un mélange de substances odorantes enrichi en diastéréoisomères selon l'une des revendications 1 ou 2 précédentes, la distillation fine du procédé de distillation s'effectuant dans un processus continu.

4. Procédé de fabrication d'un mélange de substances odorantes enrichi en diastéréoisomères selon l'une des revendications 1 à 3 précédentes, la distillation fine du procédé de distillation présentant un rapport de reflux d'au moins 5 : 1.

5. Procédé de fabrication d'un mélange de substances odorantes enrichi en diastéréoisomères selon l'une des revendications 1 à 4 précédentes, la distillation fine du procédé de distillation étant effectuée à des températures comprises entre 120 °C et 150 °C.

6. Procédé de fabrication d'un mélange de substances odorantes enrichi en diastéréoisomères selon l'une des revendications 1 à 5 précédentes, la distillation fine du procédé de distillation étant effectuée à une pression réduite d'environ 1 à 100 mbar.

7. Procédé de fabrication d'un mélange de substances odorantes enrichi en diastéréoisomères selon l'une des revendications 1 à 6 précédentes, dans lequel la première étape de distillation est une distillation en couche mince.

8. Procédé de préparation d'un mélange de substances odorantes enrichi en diastéréoisomères selon la revendication 7, dans lequel la distillation en couche mince comprend deux étapes :
- la séparation du solvant à une pression réduite d'environ 1 mbar à 400 mbar ; et
- l'extraction du produit brut comprenant des composés de formule générale (A) à une pression réduite d'environ 0 mbar à 100 mbar.

9. Procédé de préparation d'un mélange de substances odorantes enrichi en diastéréoisomères selon l'une des revendications 7 ou 8 précédentes, dans lequel la première étape de la distillation en couche mince est effectuée à une température d'enveloppe comprise entre 120 °C et 200 °C et dans lequel la deuxième étape de la distillation en couche mince est effectuée à une température d'enveloppe comprise entre 150 °C et 250 °C.

10. Mélange de substances odorantes enrichi en diastéréoisomères comprenant des composés de formule générale (A) : 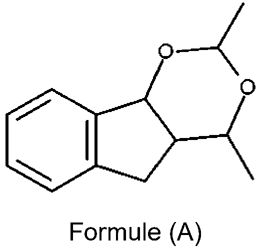
les composés de formule (A) comprenant les paires d'énantiomères diastéréoisomères suivants de formules (I), (II) et (III) : 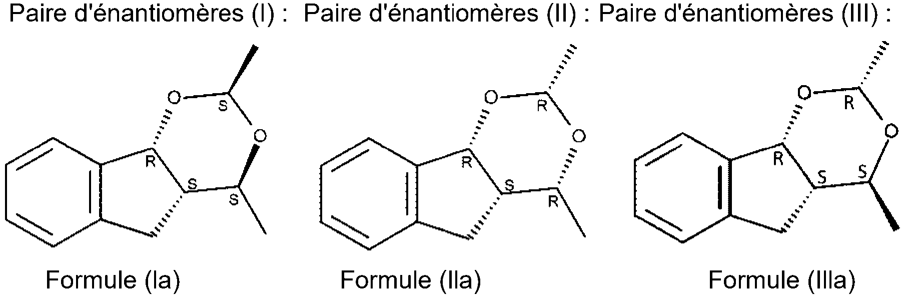 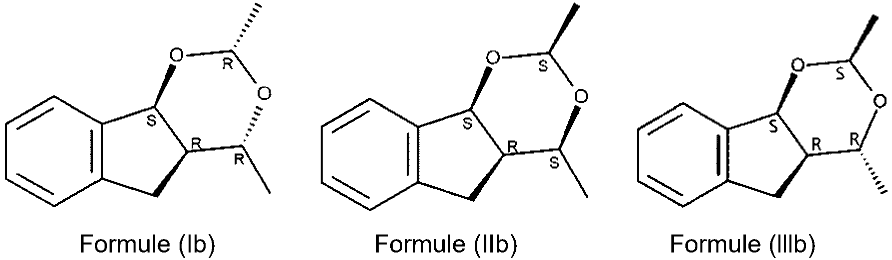
le rapport quantitatif entre la paire d'énantiomères (I) et la paire d'énantiomères (II) étant d'au moins 10 : 1 ; et
le rapport quantitatif entre les paires d'énantiomères (I) et (III) étant d'au moins 50 : 1.

11. Mélange de substances odorantes enrichi en diastéréoisomères selon la revendication 10, le mélange de substances odorantes comprenant au total au moins 96,5 % en poids des paires d'énantiomères (I), (II) et (III).

12. Utilisation du mélange de substances odorantes enrichi en diastéréoisomères selon l'une des revendications 10 ou 11 précédentes comme substance odorante ou pour la fabrication d'une préparation odorante.

13. Préparation odorante contenant une quantité sensoriellement efficace du mélange de substances odorantes enrichi en diastéréoisomères selon l'une des revendications 10 ou 11 précédentes.

14. Utilisation du mélange de substances odorantes enrichi en diastéréoisomères selon l'une des revendications 10 ou 11 précédentes en une quantité sensoriellement efficace pour conférer une note florale à un produit parfumé, modifier ou renforcer cette note florale, ou pour la fabrication d'un produit parfumé.

15. Produit parfumé comprenant le mélange de substances odorantes enrichi en diastéréoisomères selon la revendication 10 ou 11 ou une préparation de substances odorantes selon la revendication 13.
